# EUROPEAN PATENT SPECIFICATION

(11) **EP 1 370 291 B1**
(45) Date of publication and mention of the grant of the patent: **16.06.2010**
(21) Application number: 02725145.3
(22) Date of filing: 15.03.2002
(51) Int. Cl.: A61K 41/00, A61N 5/06, A61B 19/00, A61B 5/00, G01N 21/64, A61B 1/04, G02B 21/00

(54) **DEVICE AND METHOD FOR THE PHOTODYNAMIC DIAGNOSIS OF TUMOR TISSUE**
VORRICHTUNG UND VERFAHREN ZUR PHOTODYNAMISCHEN DIAGNOSE VON TUMORGEWEBE
DISPOSITIFS CHIRURGICAUX TELESCOPIQUES

(30) Priority: 16.03.2001 US 276036 P; 30.10.2001 US 336316 P
(43) Date of publication of application: 17.12.2003
(73) Proprietor: University of Utah Research Foundation, Salt Lake City UT 84108 (US)
(72) Inventor: MCGREEVY, James, Salt Lake City, UT 84121 (US); GRISSOM, Charles, B., Salt Lake City, UT 84112 (US); CANNON, Michelle, St Louis, Missouri 63108 (US)
(74) Representative: Gowshall, Jonathan Vallance
(86) International application number: PCT/US2002/007699
(87) International publication number: WO 2002/074339

(56) References cited:
- EP-A- 0 280 397
- EP-A- 0 805 348
- WO-A-01/30967
- WO-A-97/11636
- DE-A- 4 133 493
- US-A- 5 772 580
- US-A- 5 912 735

## Description

The present disclosure relates to devices and methods for exciting a biologically-targeted fluorescent molecule with light from a light source (e.g., a laser). According to certain embodiments, fluorescent cobalamins (sometimes referred to herein as CobalaFluors) can be used with the disclosed devices and methods. The fluorescent cobalamins comprise a fluorescent, phosphorescent, luminescent or light producing compound that is covalently linked to cobalamin. These fluorescent cobalamins can be used as diagnostic and prognostic markers (a) to distinguish cancer cells and tissues from healthy cells and tissues, including identifying lymph nodes containing cancer cells, and (b) to determine if an individual will respond positively to chemotherapy using cobalamin-based therapeutic bioconjugates.

### BACKGROUND

The publications and other materials cited herein are referenced in the following text by author and date and are listed alphabetically by author in the appended bibliography.

Rapidly-dividing cells require cobalamin as a cofactor for the enzyme methionine synthase to support one-carbon metabolism prior to DNA replication (Hogenkamp et al., 1999). In acute promyelocytic leukemia, a 3-26 fold increase in the unsaturated B₁₂ binding capacity of blood is observed, due to an increase in the concentration of the B₁₂ binding proteins transcobalamin and haptocorrin (Schneider, et al., 1987; Rachimelwitz, et al., 1971). Some patients with solid tumors also exhibit a significant increase in the circulating levels of transcobalamin and haptocorrin (Carmel, et al., 1975). The increase in unsaturated serum cobalamin binding capacity corresponds to the increased uptake of cobalamin by rapidly dividing cells. Tumors even sequester sufficient cobalamin for diagnostic imaging purposes if a gamma-emitting radionuclide, such as ¹¹¹In, is attached to cobalamin through the octadentate chelator diethylenetriaminepentaacetic acid (DTPA) (Hogenkamp and Collins, 1997). This has been demonstrated in mice with an implanted fibrosarcoma (Hogenkamp and Collins, 1997), as well as in humans with breast cancer (Collins et al., 1999), and in tumors of the prostate, lung and brain (Collins et al., 2000).

In the sentinel lymph node concept for melanoma and breast cancer surgery, a dye or radionuclide is injected into the tissue around the tumor to identify the first lymph node that drains the tumor (Morton et al., 1992; McGreevy, 1998). This node is termed the sentinel node, and it is removed for diagnostic tests to determine the extent of metastasis beyond the primary tumor. This procedure is controversial, as it fails to detect metastatic disease in about 12% of patients (McMasters et al., 1999). The dye or radionuclide that is injected is not specific for cancer cells, but merely identifies for the surgeon the primary lymph node that drains the region of the tumor. The high false-negative rate should be improved dramatically by using a fluorescent marker that is specific for cancer cells.

Thus, there exists a need for an agent and instruments that can be used for the diagnosis and prognosis of cancer tissue or cells with improved results.

A device according to the preamble of claim 1 is known e.g from EP 0 805 348.

### SUMMARY OF THE DISCLOSURE

The present invention is defined in the claims.

Detection and imaging apparatuses are described that may be used to detect, manipulate and/or remove fluorescent, phosphorescent or luminescent compounds or tissue in a host. According to a first embodiment, the apparatus includes a surgical telescopic device having a distal end and a proximal end; a camera coupled to the proximal end of the surgical telescopic device; and a holographic notch filter interposed between the camera and the proximal end of the surgical telescopic device. In some embodiments, the camera is a charge-coupled-device camera ("CCD camera"). The apparatus also includes a focusing lens and optionnally an additional type of filter such as a long-pass filter or an infrared filter.

The apparatus includes a light-generating source that illuminates the fluorescent, phosphorescent or luminescent material; a surgical telescopic device having a distal end and a proximal end; a focusing lens having a first end positioned adjacent to the proximal end of the surgical telescopic device; a camera coupled to a second end of the focusing lens; and at least one member interposed between the proximal end of the surgical telescopic device and the camera, wherein the member removes substantially only the wavelength transmitted by the light-generating source. The member may be a filter, prism, grating, mirror, or similar wavelength selection device.

In a further disclosed embodiment, the apparatus includes a housing that includes a magnifying lens; a holographic notch filter interposed between the magnifying lens and the host; and a light-generating source for illuminating the fluorescent, phosphorescent or luminescent material.

The surgical telescopic device may be used by illuminating the material with non-white light and detecting the emitted fluorescence, phosphorescence or luminescence.

According to one embodiment, fluorescent cobalamins comprised of a fluorescent, phosphorescent, luminescent or light-producing compound that is covalently linked to cobalamin can be used in conjunction with the above-described apparatuses. These fluorescent cobalamins can be used as a diagnostic and prognostic marker (a) to distinguish cancer cells and tissues from healthy cells and tissues, including identifying lymph nodes containing cancer cells, and (b) to determine if an individual will respond positively to chemotherapy using cobalamin-therapeutic bioconjugates. The fluorescent cobalamins offer the properties of (1) rapid transport and storage by cancer cells (maximum uptake occurs at 4-6 hours), (2) a bright fluorophore that can be visually detected at very low concentrations, and (3) nontoxic components.

In one aspect, fluorescent cobalamins are provided in which fluorescent, phosphorescent, luminescent or light-producing compounds are covalently linked to cobalamin (vitamin B₁₂).The fluorescent, phosphorescent or light-producing compounds can be covalently linked to the cobalt atom, the corrin ring, or the ribose moiety of cobalamin. It is preferred to covalently link the fluorescent, phosphorescent, luminescent or light-producing compound to the corrin ring or the ribose moiety. Although, any fluorescent, phosphorescent, luminescent or light-producing compound can be utilized in preparing the fluorescent cobalamins, it is preferred to utilize fluorescent, phosphorescent, luminescent or light-producing compounds that are excitable with visible or infrared light. Examples of preferred fluorescent compounds include, but are not limited to, fluorescein, fluorescein-5EX, methoxycoumarin, naphthofluorescein, BODIPY 493/503, BODIPY FL, BODIPY R6G, BODIPY 530/550, BODIPY TMR, BODIPY 564/570, BODIPY 576/589, BODIPY 581/591, BODIPY TR, Cascade Blue, Dansyl, Dialkylaminocoumarin, 4',5'-dichloro-2',7'-dimethyoxyfluorescein, 2',7'-dichlorofluorescein, eosin, eosin F3S, erythrosin, hydroxycoumarin, lissamine rhodamine B, methosycoumarin, maphthofluorescein, NBD, Oregon Green 488, Oregon Green 500, Oregon Green 514, PyMPO, pyrene, rhodamine 6G, rhodamine green, rhodamin red, rhodol green, 2',4',5',7'-tetrabromosulfonefluorescein, tetramethylrhodamine (TMR), Texas Red, X-rhodamine, Cy2 dye, Cy3 dye, Cy5 dye, Cy5.5 dye, Cy7 dye, IC Green, or a quantum dot structure. The preferred fluorescent cobalamins fluoresce when excited by visible or infrared light without the need to separate the fluorescent or phosphorescent compound from cobalamin. The light may be provided by a laser or a fiber optic light source with appropriate filter. Red light is used for better tissue penetration.

In a second aspect, the fluorescent cobalamins are used to identify atypical cells such as neoplastic cells, dysplastic cells, or hyperplastic cells. More particularly, the fluorescent cobalamins are used to distinguish cancer cells from healthy cells. In one embodiment, a fluorescent cobalamin is administered to a patient prior to surgery. The presence of fluorescence, phosphorescence, luminescence or emitted light in cancer cells is used by the surgeon to define the tissue to be removed, whether in a primary tumor or in a metastatic site. In a second embodiment, a fluorescent cobalamin is administered to a patient in a manner suitable for uptake by lymph nodes draining the *situs* of the tumor. The presence of fluorescence, phosphorescence, luminescence or emitted light identifies those lymph nodes that should be removed during surgery. In this latter embodiment, laparoscopic, endoscopic and microscopic techniques can be utilized to identify lymph nodes with cancer cells. The use of these techniques facilitates the identification and retrieval of positive lymph nodes.

In a third aspect, the fluorescent cobalamins are used to determine if an individual will respond positively to chemotherapy using cobalamin-based therapeutic bioconjugates. In this aspect, a fluorescent cobalamin is used to assess the ability of the particular cancer cell type to transport and store cobalamin, both qualitatively and quantitatively. Various types of cancer that transport and store large amounts of cobalamin are good candidates for therapy with cobalamin-based therapeutic bioconjugates. Quantification of tumor cell cobalamin binding, uptake, transport, and storage can be carried out by fluorescence under visual inspection (e.g. tissue slide), by epifluorescence microscopy, fluorescence laparoscopy, fluorescence endoscopy or flow cytometry.

In a fourth aspect, the fluorescent cobalamins are used to determine the levels of cobalamin in blood, plasma, serum, cerebrospinal fluid or urine or to determine the amount of unbound cobalamin binding capacity in blood, plasma, serum or cerebrospinal fluid.

In a fifth aspect, any fluorescent molecule (cancer-targeted or non-targeted) can be detected in a lymph node using laparoscopic or endoscopic visualization.

### BRIEF DESCRIPTION OF THE DRAWINGS

Figure 1 shows the synthesis of one embodiment of the presently disclosed fluorescent cobalamin.
Figure 2 shows the synthesis of cobalamin monocarboxylic acids.
Figure 3 shows the conjugation of cobalamin carboxylic acids with 1,12-diaminododecane.
Figure 4 shows conjugation of fluoroscein-5EX-NHS ester with the diaminododecane cobalamin derivative.
Figure 5 shows the fluorescence emission spectrum of fluorescein-5EX-b-cobalamin derivative CBC-123.
Figure 6 shows the synthesis of CobalaFluor Y.
Figure 7 shows fluorescence emission spectrum of CobalaFluor Y (Cy5 CobalaFluor).
Figure 8 shows the immobilization of a cobalamin analog on a CM5 BIAcore chip.
Figure 9 shows a competition assay sensorgram.
Figure 10 shows the competition of cobalamin for TCII binding.
Figures 11A-11C show the Kd values for cobalamin, cobalamin analogs and CobalaFluors.
Figure 12 shows tumor imaging in animal models.
Figure 13 shows tumor imaging in neoplastic breast tissue.
Figure 14 shows tumor imaging in neoplastic lymph node tissue.
Figure 15 is a schematic representation of a telescopic surgical device, including a video monitoring and control system.
Figure 16 is a perspective view of a telescopic surgical device.
Figure 17 is a perspective view of a portion of a telescopic surgical device.
Figure 18 is a plan view of an end of a telescopic surgical device.
Figure 19 is a perspective view of surgical microscope head that includes a filter.
Figure 20 is a perspective view of telescopic device that includes a filter.
Figure 21 is a schematic representation of one embodiment of a light source for a telescopic surgical device.
Figure 22 shows sites for modification on the cobalamin molecule.

### DETAILED DESCRIPTION OF SEVERAL EMBODIMENTS

The disclosed surgical telescopic devices may be used to detect and remove fluorescent, phosphorescent or luminescent material. Such material may include the molecules and compounds described herein as well as any tissue that has been labeled with fluorescent, phosphorescent or luminescent molecules or compounds. The devices can excite a biologically-targeted fluorescent molecule with light and can detect the resulting fluoresced light that is of longer wavelength than the exciting light.

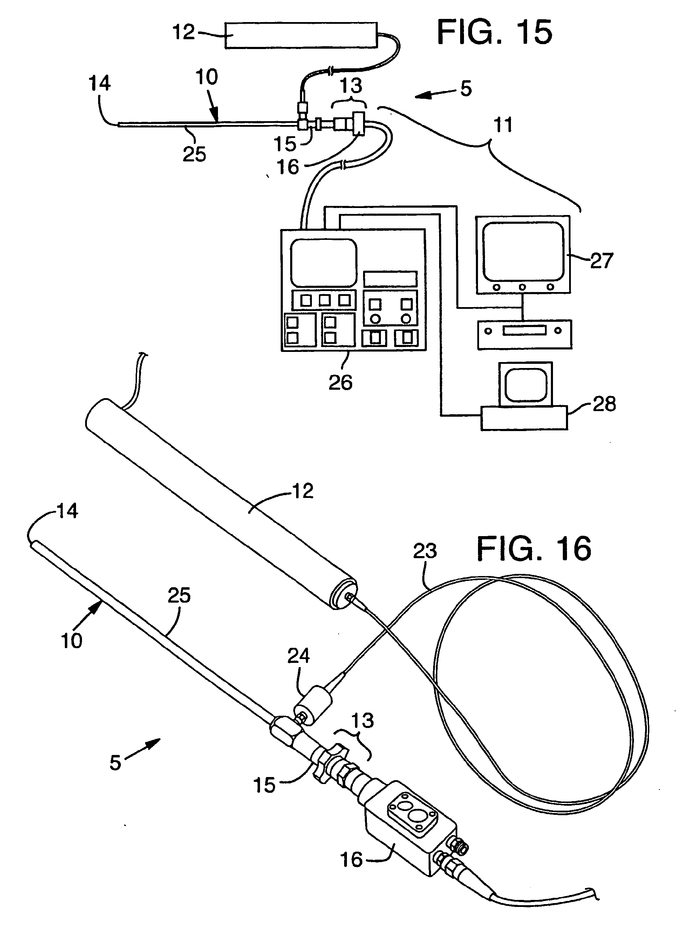

Illustrative surgical telescopic devices include, for example, an endoscope, laparoscope, arthroscope, colonoscope or microscope. The surgical telescopic device has been modified to include at least one light-removing or blocking member and a red-light illumination source. These modifications enhance visualization of fluorescent, phosphorescent or luminescent material. According to one particular embodiment, the apparatus is a laparoscope for identifying and removing axillary lymph nodes that may or may not contain cancerous cells. An example of such a laparoscope is described below in more detail in connection with FIGS. 15-18 and 21.

As mentioned above, the apparatus includes at least one light-removing member. As used herein, "light-removing" includes, but is not limited to, any light-blocking mechanism such as absorbing, reflecting, and/or deflecting a predetermined wavelength of light. Illustrative members include a filter, prism, grating, mirror, or similar wavelength selection device. An apparatus may include more than one such member and/or a combination of such members.

Suitable filters include a notch filter, a long-pass filter or an infrared filter. According to a particular embodiment, the filter is a holographic notch filter that blocks only a very narrow wavelength that corresponds to the wavelength of the light-generating source that is used to excite the fluorescent, phosphorescent or luminescent material. A holographic notch filter appears clear when viewed by the human eye. The holographic notch filter allows for the use of color CCD cameras for visualization of the procedure without repeatedly substituting different types of filters and cameras. Holographic notch filters are well-known and are commercially available, for example, from Kaiser Optical Systems.

Long-pass or band pass filters could also be used to remove shorter wavelength light, but allow longer wavelength light to pass. Long-pass filters may be useful because light produced by the fluorescence of a molecule is shifted to a longer wavelength relative to the wavelength of the light used to illuminate the fluorophore.

The filter prevents the reflected and direct incident light from the light-generating source from distorting or obscuring the image acquired by the camera. The filter is selected to remove the wavelength of the non-white light-generating source. For example, in the case of a red light laser or laser diode with a wavelength of about 625 nm a holographic notch filter that removes only light wavelength of about 625 nm is utilized. Thus, the color image obtained by the camera will not be distorted by the red light.

The light-removing member may be removably fixed in the surgical telescopic device so that a user can easily interchange between different light-removing members based on the desired viewing mode. For example, a mechanical shutter could be provided for intermittently introducing a filter in front of the camera.

The light-generating source may be any suitable device such as a laser, laser diode, a light-emitting diode, a fiber optic light source, a luminous gas discharge, a hot filament lamp, and similar light sources. The light-generating source generates red light such as, for example, light in a wavelength range of at least about 625 nm, particularly about 630 to about 635 nm, for the fluorescent cobalamin that includes Cy5 dye. An illustrative light-generating source is a HeNe laser that emits a red light with a wavelength of about 633 nm. The HeNe laser emits at least about 10 mW for better illumination of the tissue. A red light source can penetrate tissue up to several centimeters and is used to illuminate and identify the fluorophores and any associated cancer cells.

A white light source is also provided allowing for more familiar full color viewing. Such full color viewing is useful for anatomical orientation within the host and for viewing on the video monitor. A dual light source that includes both the red light source (or light of any non-white color) and the white light source is utilized to provide an easy mechanism for rapid switching between non-white light for fluorescence viewing and white light for conventional viewing. Such switching may be accomplished by any mechanism such as, for example, voice-actuated switching, a mechanically-operated switch (e.g., a foot pedal), an optically-operated switch, or an electronically-operated switch. For example, a commercially-available fiber optic dual-lamp xenon light source may be modified by replacing one of the lamps with either a red diode laser or the output of a red HeNe laser. Another variant could be a device that includes two internal light sources (one white and one non-white) and a mirror or prism under mechanical or electromechanical control to switch between the two light sources.

The camera may be any device that can detect, capture or transmit a fluorescent, phosphorescent or luminescent image. Illustrative cameras include a video camera or a photographic film camera. According to a particular embodiment, the camera is a CCD camera. Illustrative CCD cameras include those available from Santa Barbara Instrument Group (such as their camera available under the trade designation "STV"), Stryker, and Karl Storz.

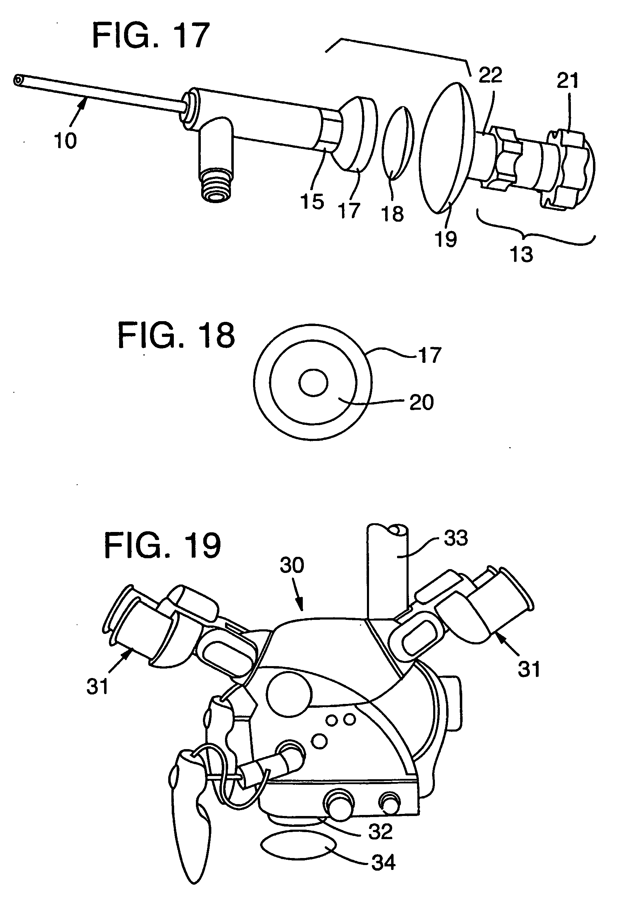

An illustrative embodiment of the apparatus is depicted in FIGS. 15, 16, and 21. Referring to FIG. 15, a surgical telescopic system **5** includes a surgical telescopic device **10** coupled to a CCD camera system **11** and to a light-generating source **12.** The surgical telescopic device **10** is a rigid or flexible tubular optical instrument having a shaft **25,** a distal end **14** that is inserted into an incision or opening in a patient, and a proximal end **15** that is coupled to the CCD camera system **11.** The surgical telescopic device **10** may be coupled to the CCD camera system **11** by any connecting structure **13** capable of transmitting the light emitted by the illuminated tissue to CCD photosensors included in the CCD camera system **11.** The connecting structure **13** is described below in more detail in connection with FIG. 17. The CCD camera system includes a camera head **16** that is mounted onto the connecting structure **13.** The camera head **16** communicates via cables with a video processing module **26** that can accept national television standards committee ("NTSC") or video helical scan ("VHS") format from the video output signals generated from the CCD photosensors in the camera head **16**. The CCD camera system **11** also includes a video monitor **27** for viewing and recording images during surgery and a computer **28** for controlling the video processing module b. Other suitable CCD camera control and viewing systems may be used with the surgical telescopic device.

Referring to FIG. 16, the light-generating source **12** is connected to the surgical telescopic device **10** via a fiber optic cable **23** received in a fitting **24** that communicates with the shaft **25** of the surgical telescopic device **10.** The light-generating source also may be connected to the surgical telescopic device **10** via an input opening in the camera head **16** for receiving a fiber optic cable. The light from the light-generating source **12** typically travels through an optical fiber or cable (not shown) disposed within the shaft **25** of the surgical telescopic device **10** to illuminate the desired host region. Alternatively, the light-generating source **12** is not coupled to the surgical telescopic device **10** but illuminates the desired host region by independent means such as an optical fiber inserted into an incision in the host.

The connecting structure **13** between the camera head **16** and the proximal end **15** of the surgical telescopic device **10** may include various components. The connecting structure **13** may be a holder for the filter that is fitted to an eyepiece of the surgical telescopic device **10** and a lens of the camera head **16.** FIG. 17 depicts a more detailed example of one embodiment of the connecting structure **13.**

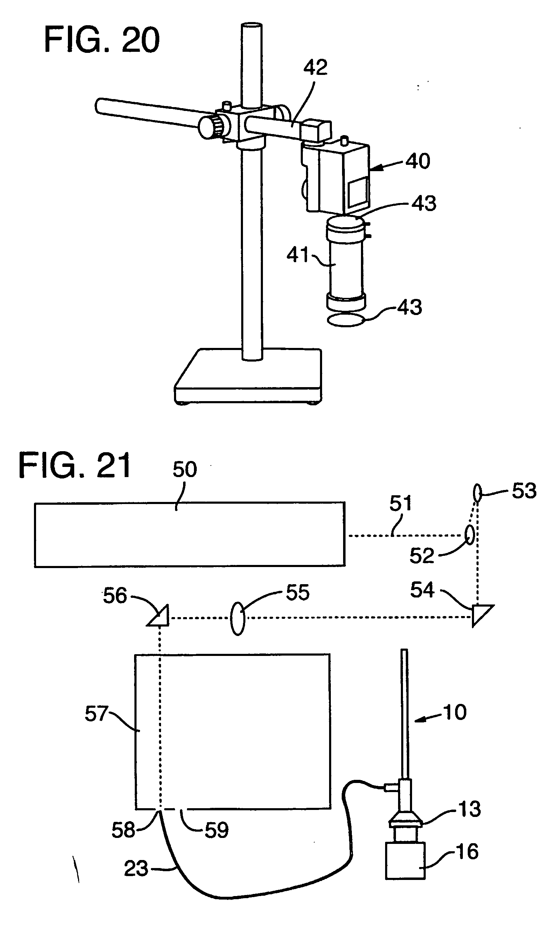

FIG. 21 shows one particular light source in more detail. A HeNe laser **50** emits a light beam **51** that reflects from a first mirror **52** and a second mirror **53,** then is deflected by a first prism **54** through a band pass filter **55** for deflection by a second prism **56** into a dual-lamp xenon light source **57.** The dual-lamp xenon light source **57** defines a first outlet **58** through which the laser light exits into the fiber optic cable **23.** The dual-lamp xenon light source **57** also defines a second outlet **59** through which white light from the xenon light source exits. The first mirror **52,** second mirror **53,** first prism **54,** band pass filter **55,** and second prism **56** may be arranged on an optical table. The first mirror **52,** second mirror **53,** first prism **54,** band pass filter **55,** and second prism **56** shown in FIG. 21 dissipate the non-coherence of the particular HeNe laser **50** and may not be required with other light sources.

Referring to FIG. 17, the proximal end **15** of the surgical telescopic device **10** is provided with a contiguous eyepiece **17.** A holographic notch filter **18** is disposed between the eyepiece **17** and a filter holder **19.** As depicted in FIG. 18, a recess **20** may be provided in the eyepiece **17** for receiving the holographic notch filter **18.** The holographic notch filter **18** typically is a circular disk but it may have any shape such as an oval, a rectangle or a square. The thickness of the holographic notch filter **18** may vary as is known in the art depending upon the desired blocking wavelength and other performance characteristics. The filter holder **19** is dimensioned so that it fits as a sleeve around the outer periphery of the eyepiece **17.** The surface of the outer periphery of the eyepiece **17** and the inner surface of the filter holder **19** may be threaded for removably engaging the eyepiece **17** with the filter holder **19.** A focusing lens **22** is mounted to the filter holder **19.** An adapter **21** (e.g., a C-mount) connects the focusing lens **22** to the CCD camera head **16** via means known in the art.

It will be appreciated that there are many other possible arrangements for locating the filter. For example, a filter may be provided between the focusing lens and the CCD camera head. Another option is to provide a filter inside the shaft of the endoscope or laparoscope. A plurality of filters may be included in a single apparatus. For example, the filter holder may hold more than one filter or a first filter may be disposed between the eyepiece and the focusing lens and a second filter may be disposed between the focusing lens and the CCD camera head. A combination of different filter types may also be used such as a combination of a notch filter, a long-pass filter and/or an infrared filter. According to one particular alternative embodiment, a first infrared filter is disposed between the eyepiece and the focusing lens, and second and third infrared filters are disposed between the focusing lens and the CCD camera head. In this embodiment, the infrared filters allow for the use of a black-and-white CCD camera that may provide better contrast for detection of the fluorophores.

Also disclosed are magnifying apparatuses that may be useful for tumor micromargin visualization. In general, such apparatuses includes a magnifying lens, an eyepiece, and a filter disposed between the host and the eyepiece of the microscope.

An example is a surgical microscope as shown in FIG. 19. The surgical microscope includes a head or housing **30** that carries two eyepieces **31.** The housing **30** is supported by an articulating arm **33** that is secured to a ceiling mount (not shown). The eyepieces **31** optically communicate with a light collecting lens **32** that receives the light reflected or emitted by the object. A holographic notch filter **34** is affixed to the light collecting lens **32** by screws, clips or similar attachment means. A fiber optic bundle (not shown) may be fixed to the articulating surgical microscope head to provide the illuminating light. An intensifier screen (not shown) may be mounted to the microscope on a separate eyepiece to provide greater detection sensitivity. After tumor debulking via lumpectomy, the surgical microscope may be positioned for magnified inspection of the tumor micromargins for fluorescence traces indicating remaining tumor.

Another variant of an instrument for tumor micromargin inspection is depicted in FIG. 20. This apparatus includes a CCD camera system **40** as described above, but a magnifying video lens **41** rather than an endoscope or laparoscope is mounted to the CCD camera head. An articulating positioning arm **42** supports the CCD camera **40.** A holographic notch filter **43** is interposed between the CCD camera **40** and the magnifying video lens **41** or it may be affixed to the distal end of the magnifying video lens **41** by a filter holder as described above. After tumor debulking via lumpectomy, the CCD camera microscope may be positioned for magnified inspection of the tumor micromargins for fluorescence traces indicating remaining tumor.

Any type of fluorescent, phosphorescent, or luminescent material could be used with the above-described devices. A particularly useful material is a fluorescent cobalamin that comprises a fluorescent compound (fluorophore), a phosphorescent compound (phosphorophore), a luminescent compound (chemiluminescent chromophore) or a light-producing compound that is covalently linked to cobalamin (vitamin B₁₂). These fluorescent cobalamins can be used as diagnostic and prognostic markers (a) to distinguish cancer cells and cancerous tissue from healthy cells and tissues, including identifying lymph nodes containing cancer cells, and (b) to determine if an individual will respond positively to chemotherapy using cobalamin-therapeutic bioconjugates.

The fluorescent cobalamins can be represented by the following formula 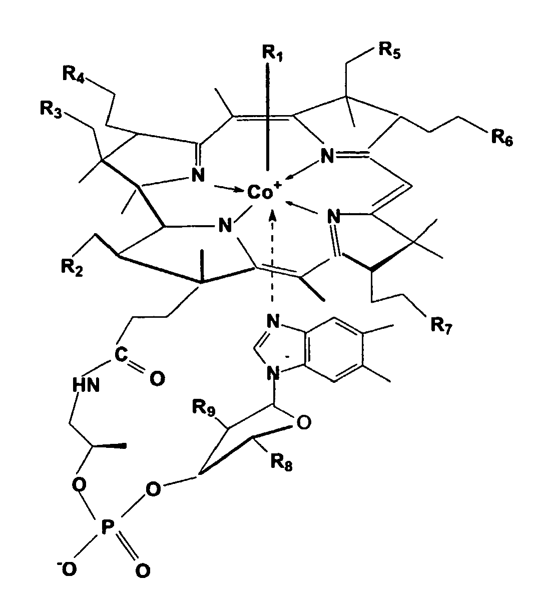 where R₁ is CN, OH, OH₂, CH₃, 5'-deoxyadenosine or (CH₂)ₚNHC(=S)Y; R₂, R₃, R₄, R₅, R₆, and R₇ are independently CONH₂ or CO-XₘY; R₈ is CH₂OH, O(C=O)XₘY or CH₂O(C=O)XₘY; R₉ is OH or O(C=O)XₘY; X is a linker having the formula N(CH₂)ₙNHO(C=O) or NH-(CH₂)ₙ-NH; Y is a fluorophore, a phosphorophore, chemiluminescent chromophore or a light-producing molecule; m is 0 or 1, n is 0-50 and p is 2-10, with the proviso that at least one of R₁ - R₉ groups contains Y.

The fluorescent cobalamins may be prepared by covalently attaching a fluorophore, a phosphorophore, chemiluminescent chromophore or a light-producing molecule to cobalamin. The fluorophore, phosphorophore, chemiluminescent chromophore or light-producing molecule is covalently linked to the cobalt atom, to the corrin ring or to the ribose sugar directly or via a linker molecule. The covalent linkage is preferably accomplished with the use of a linker molecule. If the fluorophore, phosphorophore, chemiluminescent chromophore or light-producing molecule is attached to the cobalt atom of cobalamin, the fluorescence, phosphorescence or emitted light is diminished in intensity through quenching by the spin of the cobalt atom. In addition, prolonged exposure of the fluorescent cobalamin to light will cleave the cobalt-carbon bond and release the fluorophore, phosphorophore, chemiluminescent chromophore or light-producing molecule from cobalamin (Howard et al., 1997). Thus, it is preferred to attach the fluorophore, phosphorophore, chemiluminescent chromophore or light-producing molecule to the corrin ring or the ribose moiety of the cobalamin molecule. These latter fluorescent cobalamins do not have the disadvantages of the fluorescent cobalamins in which the fluorophore, phosphorophore, chemiluminescent chromophore or light-producing molecule is covalently linked to the cobalt atom.

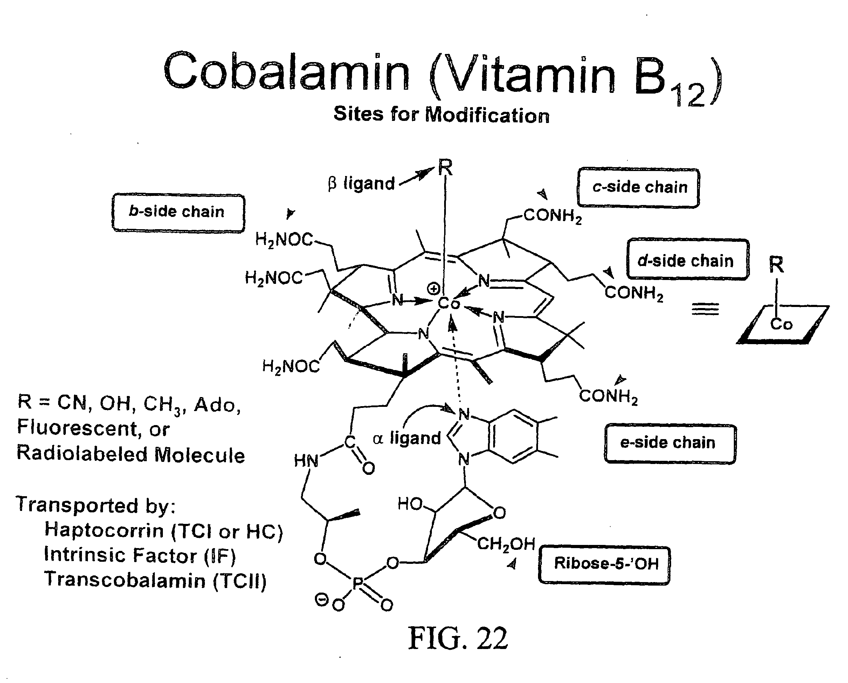

Attachment of the fluorophore, phosphorophore, chemiluminescent chromophore or light-producing molecule to a carboxylate on the corrin ring or the 5'-ribose hydroxyl group circumvents the problem of lower sensitivity and photolability. In general, corrin ring carboxylate derivatives (Collins and Hogenkamp, 1997) are known, but none of the compounds synthesized have contained a fluorescent marker. The fluorophore, phosphorophore, chemiluminescent chromophore or light-producing molecule can be attached directly to the corrin ring, rather than to the cobalt atom by derivatization of the cobalamin monocarboxylate according to published methods (Collins and Hogenkamp, 1997 and references cited therein). Figure 22 shows sites on cobalamin which can be used for modification.

Although, any fluorophore, phosphorophore, chemiluminescent chromophore or light-producing molecule can be utilized in preparing the fluorescent cobalamins, it is preferred to utilize fluorophores that are excitable with visible or infrared light. It is preferred to use visible or infrared light for in vivo use of the fluorescent cobalamins. Examples of preferred fluorophores include, but are not limited to, fluorescein, fluorescein-5EX, methoxycourmarin, naphthofluorescein, BODIPY 493/503, BODIPY FL, BODIPY R6G, BODIPY 530/550, BODIPY TMR, BODIPY 564/570, BODIPY 576/589, BODIPY 581/591, BODIPY TR, Cascade Blue, Dansyl, Dialkylaminocoumarin, 4',5'-dichloro-2',7'-dimethyoxyfluorescein, 2',7'-dichlorofluorescein, eosin, eosin F3S, erythrosin, hydroxycoumarin, lissamine rhodamine B, methosycoumarin, maphthofluorescein, NBD, Oregon Green 488, Oregon Green 500, Oregon Green 514, PyMPO, pyrene, rhodamine 6G, rhodamine green, rhodamin red, rhodol green, 2',4',5',7'-tetrbromosulfonefluorescein, tetramethylrhodamine (TMR), Texas Red, X-rhodamine, Cy2 dye, Cy3 dye, Cy5 dye, Cy5.5 dye, Cy7 dye, IC Green, or a quantum dot structure. The preferred fluorescent cobalamins fluoresce when excited by visible or infrared light without the need to cleave the fluorophore from the bioconjugate. The light may be provided by a laser or a fiber optic light source with an appropriate filter. Red light is preferred for better tissue penetration.

It has been found that there is differential uptake of fluorescent cobalamin analogues in normal and leukemic human bone marrow. The difference between normal marrow cells and leukemic myeloblasts (cancer cells) is particularly noteworthy, with no detectable cobalamin being taken up by normal cells. Bone marrow samples from healthy individuals show no fluorescent labeling. It has also been found that there is uptake of a doxorubicin-cobalamin conjugate, originally synthesized as a potential chemotherapeutic compound. Cellular uptake of the doxorubicin-cobalamin conjugate can be observed in P-388 murine leukemia cells, as well as in HCT-116 human colon tumor cells. Thus, the uptake of fluorescent derivatives of cobalamin occurs in leukemia and solid tumor cell lines. These results, in combination with the knowledge that all cancer cells increase cobalamin transport and storage, demonstrate the general applicability of the use of fluorescent cobalamins to distinguish cancer cells from normal cells.

Thus, the fluorescent cobalamins can be used to:
- identify cancerous tissue visually, via fluorescence microscopy, fluorescence laparoscopy, fluorescence endoscopy, or flow cytometry;
- identify cancerous cells in tissue sections or samples from tissue biopsies;
- define tumor margins *in vivo, ex vivo* or *in situ;*
- diagnose, detect, prognose, predict or monitor cancer *in vivo, ex vivo* or *in situ;*
- identify metastatic cancer *in vivo*, *ex vivo* or *in situ;*
- determine the stage of cancer progression;
- identify cancer dermally or transdermally;
- identify metastatic cancer dermally or transdermally;
- identify cancer in lymph nodes, including in the sentinel lymph node or nodes or in an axillary lymph node or nodes, including with the use of minimally invasive techniques, such as laparoscopy or endoscopy;
- identify metastatic disease in the treatment, detection, prediction, prognostication or monitoring of cancer, such as breast cancer, ovarian cancer, lung cancer, prostate cancer, epithelial cancer (adenocarcinoma), liver cancer, melanoma and lymphoma;
- conduct flow cytometry studies of bone marrow aspirates or peripheral blood samples for diagnosing, predicting, prognosticating, monitoring or characterizing leukemia or lymphoma;
- predict whether a patient will respond positively to chemotherapy that is based on the use of a cobalamin-therapeutic bioconjugate;
- improve the definition of tumor micromargins in a biopsy or lumpectomy;
- decrease the chance of leaving cancerous cells behind in a biopsy, lumpectomy, or tumorectomy and thereby reduce the need for follow-up surgery to remove the remaining cancer cells.

Prediction refers to understanding the biological behavior of the tumor, and how the tumor will respond (favorably or unfavorably) to therapy. Prognosis refers to the anticipated patient outcome following therapy (i.e. what is the likelihood of five- or ten-year survival following therapy). Monitoring refers to determining the success of therapy and detection of residual disease following treatment. An example is the use of a fluorescent cobalamin conjugate to test the bone marrow for the presence of myeloblasts following treatment of leukemia. Characterization refers to a descriptive or quantitative classification of the type of tumor in comparison to closely related types of tumors.

The fluorescent cobalamins can be administered in accordance with customary cancer diagnostic, detection, prediction, prognostication, monitoring or characterization methods known in the art. For example, the fluorescent cobalamins can be administered intravenously, intrathecally, intratumorally, intramuscularly, intralymphatically, or so orally. Typically, an amount of the fluorescent cobalamin of the present invention will be admixed with a pharmaceutically acceptable carrier. The carrier may take a wide variety of forms depending on the form of preparation desired for administration, e.g., oral, parenteral, intravenous, intrathecal, intratumoral, circumtumoral, and epidural. The compositions may further contain antioxidizing agents, stabilizing agents, preservatives and the like. Examples of techniques and protocols can be found in *Remington's Pharmaceutical Sciences.* The amount of fluorescent cobalamin to be administered will typically be 1-500 mg.

As shown herein, cobalamin analogs are recognized by cobalamin transport proteins, such as haptocorrin (TCI or HC), intrinsic factor (IF) or transcobalamin (TCII), with high affinity. The attachment of large molecules to cobalamin does not appear to affect protein binding.

An improvement in the surgeon's ability to identify metastatic disease in lymph nodes will advance surgical therapy by preserving, e.g., healthy tissue and minimizing the number of axillary lymph nodes removed. This will improve the patient's quality of life and improve morbidity and long-term mortality. Precise identification of cancer cells that have spread to lymph nodes will allow removal of only the diseased ducts and nodes, while sparing the healthy axillary nodes. With 186,000 new cases of breast cancer each year, the number of surgeries to remove primary tumors and determine the status of associated lymph nodes is significant. The perfunctory removal of all axillary lymph nodes and ducts leads to local edema and increased morbidity. The non-removal of axillary lymph nodes and ducts that contain metastatic cancer cells leads to decreased survival and increased long-term mortality.

In the sentinel lymph node biopsy approach, a blue dye and/or radioactive tracer are injected into the breast near the tumor. A small incision is made under the arm to look for traces of the dye or radioactivity to identify the lymph node(s) that drain the area of the breast and, as a consequence, are most likely to contain metastatic cancer cells. The above-described fluorescent cobalamin replaces the blue dye and radioisotope tracer currently used in sentinel lymph node biopsies. The use of the fluorescent cobalamins enables the application of the sentinel lymph node biopsy approach to all types of cancer. In addition, the fluorescent cobalamins enable the use of minimally invasive techniques, such as laparoscopic, endoscopic and microscopic techniques, in the analysis of cancer, especially the analysis of cancer cells in lymph nodes. The use of the fluorescent cobalamins will facilitate the identification and retrieval of positive lymph nodes. Thus, the fluorescent cobalamins can be used with the following cancers or cancers of: breast, skin (melanoma), gynecological (ovarian, prostate, uterine, cervical, vulval, penal, testicular), head and neck (lip, tongue, mouth, pharynx), digestive organs (esophageal, stomach, small intestine, large intestine, rectum, colon, liver, pancreas), bone, connective tissue, urinary organs (bladder, kidney), eye, brain and central nervous system, endocrine glands (thyroid), lymph tissues, Hodgkin's disease, non-Hodgkin's lymphoma and multiple myeloma.

In addition, the use of fluorescent cobalamins enables the use of minimally invasive techniques, such as laparoscopic and endoscopic techniques, for the identification of lymph nodes which contain cancer cells and which must be removed. The fluorescent cobalamins also may emit sufficiently bright light (e.g., bright blue in the case of CobalaFluor Y) that they can be visually detected with an unaided eye under white light. This proposed technology is designed to replace the two current methods of surgically examining the axillary lymph nodes in patients with operable breast cancer with a more accurate and less painful method. The two operations now in use are the standard axillary node dissection using a large incision (approximately 5 inches) and removing all of the lower level lymph nodes (10-15). The second, and currently experimental method, is the sentinel lymph node biopsy. This method uses either a visual dye or a gamma emitter to identify the first lymph node to drain the breast. This requires a similarly large incision and a technically challenging examination of the lymphatic pathways. The presently disclosed cobalamin molecules will take a photophore to the nodes with cancer. The lymph nodes are examined directly through three small incisions (3-5 mm) using laparoscopic instruments. The closed operative technique provides a dark field for laser excitation. The bright emission of stimulated light from the cobalamin-photophore conjugate in the tumor bearing lymph nodes will facilitate identification and retrieval of positive lymph nodes. This method will result in less dissection, less pain and better accuracy. Similar principles apply to using the fluorescent cobalamins to detect cancer cells with endoscopic techniques.

A further advantage of the fluorescent cobalamins is that they do not substantially pass through the lymphatic ducts. The two blue dyes conventionally used for sentinel lymph node procedures (Lymphazurine™ and methylene blue) tend to flow out of the lymphatic ducts into the surrounding tissue very quickly after they are injected into the tissue that drains the lymphatic ducts. Such leakage obscures the operative field with a generalized blue color.

Furthermore, since the fluorescent cobalamins are differentially taken up by cancer cells, these fluorescent cobalamins are an improved marker that will allow surgeons to excise cancerous tissue selectively, thereby leaving healthy tissue.

The ability of fluorescent cobalamins bound to cancer cells to be detected laparoscopically or endoscopically demonstrates that fluorescent molecules can be used to determine a sentinal lymph node laparoscopically or endoscopically. Thus, any fluorescent molecule (cancer-targeted or non-targeted) can be detected in a lymph node using laparoscopic or endoscopic visualization. As an example, a red fluorophore could be injected intratumorally as is now done in the sentinel lymph node procedure. Insufflation of the axilla would allow the surgeon to find the fluorescent node laparoscopically (through 2 small incisions) and thereby avoid the use of a non-cancer cell-specific radioactive tracer to help the surgeon find the general location of the sentinel node.

The fluorescent cobalamins offer several improvements as an intraoperative marker. These improvements include:
- The fluorescent marker will be specific for cancer cells in lymph ducts and nodes, rather than simply indicating which node is draining the tidal basin. The fluorescent marker will also distinguish cancer cells from healthy cells.
- The marker can be used in low concentrations because of the inherent sensitivity afforded by fluorescence detection. The blue dye now in use tends to obscure the active node and complicates postsurgical examination of the tissue by a pathologist. The blue dye also tends to obscure bleeding vessels, thereby complicating surgical excision of the node and subsequent wound closure. The use of a fluorescent marker should avoid these problems.
- A fluorescent marker that is specific for cancer cells will improve the false-negative rate of 12%, as is seen with the procedure as currently practiced.
- A decreased false-negative rate would improve the acceptance of this technique by patients and surgeons. This might decrease the training time necessary (typically 30 or more cases with complete axial node dissection) for a surgeon to learn this procedure.
- The fluorescent marker enables the use of laparoscopic, endoscopic and microscopic techniques for the visualization of cancer cells. These techniques can also be used to visualize primary tumors, metastatic tumors, axillary lymph nodes, inguinal lymph nodes and cervical lymph nodes. These techniques will reduce the necessity for large incisions and technically challenging examination of lymphatic pathways in the analysis of cancer. These techniques will result in less dissection, less pain and better accuracy.

The fluorescent cobalamins can also be used in a competitive binding assay to determine the concentration or amount of naturally-occurring cobalamin (hydroxocobalamin, methylcobalamin, adenosylcobalamin, or cyanocobalamin) in blood, plasma, serum, or other bodily fluids. In this type of assay, a fluorescent cobalamin is used in place of radioactively-labeled cobalamin in a competitive binding assay, well known to a skilled artisan. Radioactive assays for cobalamin have been described in U.S. Patent Nos. 6,096,290; 5,614,394; 5,227,311; 5,187,107; 5,104,815; 4,680,273; 4,465,775; 4,355,018, among others. This assay procedure can be used to determine the amount of unsaturated cobalamin binding capacity in blood, plasma, serum, or bodily fluids, as well as the concentration of cobalamin that is bound to the proteins transcobalamin, haptocorrin, or intrinsic factor. The use of fluorescent cobalamins has a significant advantage over radioactively-labeled cobalamin in a clinical chemistry binding assay because it does not require the special shipping, handling, and disposal procedures associated with radioactively-labeled cobalamin.

### EXAMPLES

The following Examples are offered by way of illustration and are not intended to limit the appended claims in any manner. Standard techniques well known in the art or the techniques specifically described below were utilized.

### EXAMPLE 1

### Synthesis of Fluorescent Cobalamin by Attachment of the Fluorophore to Cobalt

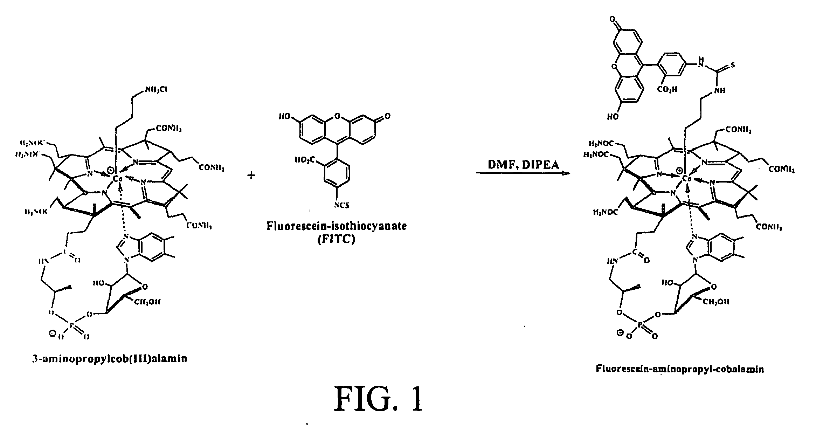

As a visual indicator of cobalamin localization, five fluorescent analogues of cobalamin were prepared by covalently attaching fluorescein to cobalamin. Under green light illumination, the fluorescein molecule emits yellow light that can be detected by the dark-adapted eye to concentrations lower than 0.1 ppm. This emission enables the sensitive detection of cancer cells via epifluorescence microscopy, as well as by visual inspection. Each of the five fluorescent cobalamins exhibited intrinsic fluorescence. All of these compounds were synthesized by reacting aminopropyl chloride with cob(I)alamin to produce aminopropylcob(III)alamin in accordance with published techniques. In a subsequent step, aminopropylcob(III)alamin was reacted with a variety of fluorophore isothiocyanates (i.e. fluorescein isothiocyanate, "FITC") to produce the corresponding fluorophore that is linked to cobalamin through an aminopropyl linker (i.e. fluorescein-aminopropyl-cob(III)alamin) This latter reaction is shown in Figure 1.

In a similar manner, fluorescent cobalamins were prepared in which the fluorophore is naphthofluorescein or Oregon Green. All the fluorescent cobalamins were found to retain high affinity for recombinant transcobalamin (rhTCII), thus allowing for a biological distribution similar to that observed for naturally occurring cobalamin.

### EXAMPLE 2

### Uptake of Cobalamin Bioconjugates by Cancer Cells

A leukemic myeloblast preparation was made from a bone marrow aspirate of a 61-year old patient having acute myelogenous leukemia (AML) M1 (minimally mature myeloblasts in the FAB classification). Cells were treated three days post-harvest with a fluorescent cobalamin prepared as described in Example 1. Differential uptake of fluorescent cobalamin analogues, as determined by fluorescence microscopy or fluorescence flow cytometry, in normal and leukemic human bone marrow cells was found. The difference between normal marrow cells and leukemic myeloblasts (cancer cells) is particularly noteworthy, with no detectable cobalamin being taken up by normal cells. A bone marrow sample from a healthy individual showed no fluorescent labeling. Uptake of a doxorubicin-cobalamin conjugate, originally synthesized as a potential chemotherapeutic compound, was seen in P-388 murine leukemia cells and in HCT-116 human colon tumor cells. These results illustrate the uptake of fluorescent derivatives of cobalamin in leukemia and solid tumor cell lines.

### EXAMPLE 3

### Preparation of Cyanocobalamin Monocarboxylic Acids

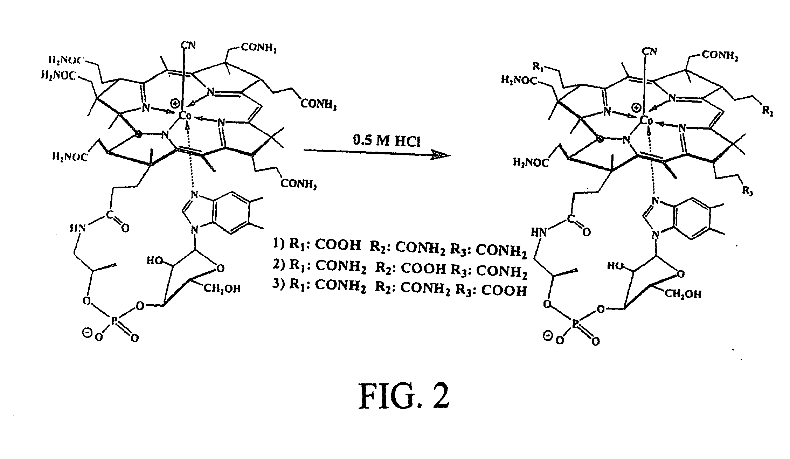

The b-, d-, and e-monocarboxylic acids were prepared by acid-catalyzed hydrolysis of cyanocobalamin. See Figure 2. Briefly, cyanocobalamin (527.0 mg, 0.389 mmol) was placed into a 100 ml round bottom flask and dissolved in 40 ml of 0.5 M HC1. The flask was placed in a water bath at 50°C and stirred for 4 hours. The reaction was monitored via HPLC (Waters, Inc. 3.9 x 300mm DeltaPak 100 C-18 column) using the gradient tabulated in Table 1.

**TABLE 1**

| Time (min) | Flow Rate (ml/min) | 0.5 M H₃PO₄ (pH 3.0 w/NH₃OH) | 9:1 CH₃CN:H₂O |
|---|---|---|---|
| 0.0 | 2.0 | 90.0 | 10.0 |
| 2.0 | 2.0 | 90.0 | 10.0 |
| 18.0 | 2.0 | 83.7 | 16.3 |
| 23.0 | 2.0 | 30.0 | 70.0 |
| 25.0 | 2.0 | 30.0 | 70.0 |
| 30.0 | 2.0 | 90.0 | 10.0 |

After 4 hours the reaction was cooled to room temperature. The pH was adjusted to 7.0 with NaOH (10%) using a pH meter. The crude material was desalted using a C-18 SepPak column (Waters, Inc. PIN WATO23635) by first rinsing the column with 10 ml methanol followed by 15 ml deionized H₂O. The crude material was applied to the column via a syringe and rinsed with 10-15 ml deionized H₂O followed by elution with 10 ml methanol. The methanol was removed via rotary evaporation and a red compound was obtained (5016-12-33).

The crude reaction mixture was dissolved in minimal deionized H₂O and half of the solution was injected onto a semi-preparative HPLC (Waters, Inc. 25.0x300mm 100 C-18 column) using the gradient calculated in Table 2.

**TABLE 2**

| Time (min) | Flow Rate (ml/min) | 0.5 M H₃PO₄ (pH 3.0 w/NH₃OH) | 9:1 CH₃CN:H₂0 |
|---|---|---|---|
| 0.0 | 40.0 | 90.0 | 10.0 |
| 4.1 | 40.0 | 90.0 | 10.0 |
| 37.0 | 40.0 | 83.7 | 16.3 |
| 47.3 | 40.0 | 30.0 | 70.0 |
| 51.4 | 40.0 | 30.0 | 70.0 |
| 61.6 | 40.0 | 90.0 | 10.0 |

Peaks at 28.0 minutes (b-monocarboxylic acid, CBC-195), 30.1 minutes (d-monocarboxylic acid, CBC-226) and 34.6 minutes (e-monocarboxylic acid) were collected using large test tubes. The pure fractions were diluted 1:1 with deionized H₂O and desalted in the same method above. In all cases, a red solid was obtained.

CBC-195 (b-monocarboxylic acid): In the two preparative runs, 74.8 mg of the b-monocarboxylic acid (14.4 %) was isolated. A positive-ion electrospray mass spectrum (ES⁺) was obtained that shows a M+1 peak (1356) and a M+22 peak (1378) as expected. The b-monocarboxylic acid (CBC-195) was obtained in an overall yield of 14%.

CBC-226 (d-monocarboxylic acid): In the two prep. runs, 38.6 mg of the d-monocarboxylic acid (7.3%) was isolated. A positive-ion electrospray mass spectrum (ES⁺) was obtained showing a M+1 peak (1356) and the corresponding M+Na peak (1378) as expected. The d-monocarboxylic acid (CBC-226) was obtained in an overall yield of 7%.

The e-monocarboxylic acid was isolated, ∼78 mg in an overall yield of 14%.

### EXAMPLE 4

### Conjugation of CNCb1 Acids with 1, 12 Diaminododecane

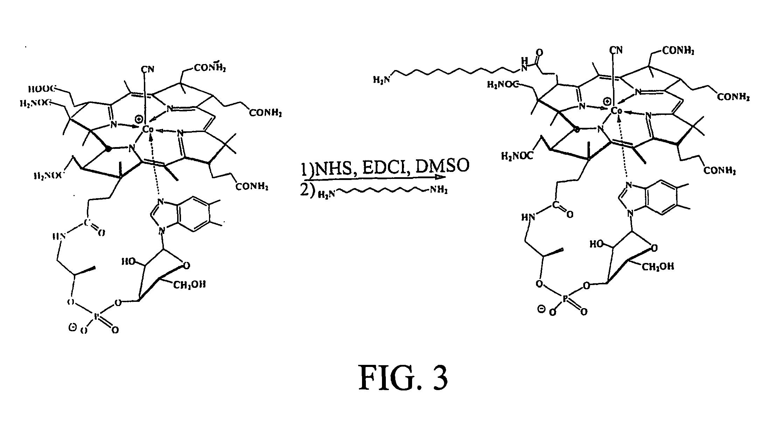

The b- and d- amides were prepared as shown in Figure 3. CBC-195 (55.4 mg, 0.0408 mmol) was added to a small glass vial and dissolved in ∼2.5 ml of DMSO followed by the addition of EDCIHC1 (12mg, 0.0626 mmol) and N-hydroxysuccinimide (NMS) (25 mg, 0.217 mmol). The reaction was stirred at room temperature overnight. From previous attempts, several equivalents of EDCI and NHS (a total of 6 equivalents) were required to drive the reaction to completion. After 24 hours, one additional equivalent of EDCI was added and the reaction was complete in a total of 26 hours. The reaction was monitored via HPLC using the gradient in Table 3. CBC-195 has a retention time of 9.07 minutes and the NHS-ester of CBC-195 has a retention time of 10.55 minutes.

**TABLE 3**

| Time (min) | Flow Rate (ml/min) | 0.5 M H₃PO₄ (pH 3.0 w/NH₃OH) | 9:1 CH3CN:H₂0 |
|---|---|---|---|
| 0.0 | 2.0 | 90.0 | 10.0 |
| 2.0 | 2.0 | 90.0 | 10.0 |
| 20.0 | 2.0 | 55.0 | 45.0 |
| 25.0 | 2.0 | 9.0 | 10.0 |

In a separate glass vial, 1,12-diaminododecane (81.8 mg, 0.408 mmol) was dissolved in ∼2 ml DMSO. The above reaction mixture was added dropwise using a syringe pump at 4.0 ml/hr to minimize dimerization. The product was formed immediately and has a retention time of 14.56 minutes. The crude reaction mixture was added to 100 ml of 1:1 CH₂Cl₂:Et₂O and a red precipitate formed. The red compound was filtered using a glass frit and washed with two 20 ml portions of CH₂Cl₂, two 20 ml portions of acetone, and finally by two 20 ml portions of Et₂O.

The crude reaction product was dissolved in a minimal amount of deionized H₂O and the solution was injected onto a semi-preparative HPLC (Waters, Inc., 25.0x100mm 100 C-18 column) using the gradient calculated in Table 4.

**TABLE 4**

| Time (min) | Flow Rate (ml/min) | 0.5 M H₃PO₄ (pH 3.0 w/NH₃OH) | 9:1 CH₃CN:H₂0 |
|---|---|---|---|
| 0.0 | 40.0 | 90.0 | 10.0 |
| 2.0 | 40.0 | 90.0 | 10.0 |
| 13.7 | 40.0 | 55.0 | 45.0 |
| 17.1 | 40.0 | 90.0 | 10.0 |

The peak at 8.70 minutes (b-amine, CBC-208) was collected using large test tubes. The pure fractions were diluted 1:1 with distilled H₂O and desalted using a C-18 SepPak column (Waters, Inc. P/N WATO23635) by first rinsing the column with 10 ml methanol followed by 15 ml deionized H₂O. The pure material was applied to the column via a syringe and rinsed with 10-15 ml deionized H₂O followed by elution with 10 ml methanol. The methanol was removed via rotary evaporation and 6 mg of a red compound was obtained.

CBC-208 (b-amine): A total of 6.0 mg of the b-amine was isolated. A positive-ion electrospray mass spectrum (ES⁺) was obtained that shows a M+1 peak (1538) and a M+23 peak (1560) as expected. CBC-208 was obtained in a yield of 9.5% after purification.

CBC-226 (d-amine): The d-monocarboxylic acid has an HPLC retention time of 9.32 minutes, the NHS-ester migrates at 10.96 minutes, and the d-amine (CBC-226) migrates at 14.93 minutes using the same HPLC gradient as in Table 3. A positive-ion electrospray mass spectrum (ES⁺) was obtained of the crude material showing a M+1 peak (1538) and the corresponding M+Na peak (1560) as expected.

### EXAMPLE 5

### Conjugation of CBC-208 and Fluorescein-5EX-NHS

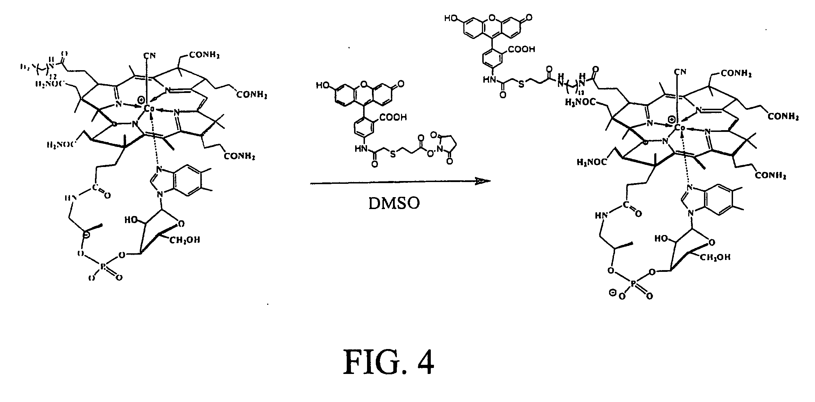

CBC-208 has been coupled to the fluorescein derivative fluorescein-5EX (available from Molecular Probes, Inc.) according to Figure 4. CBC-208 (6.0 mg, 3.87 µmol) was added to a small glass vial and dissolved in ∼0.5 ml of DMSO followed by the addition of fluorescein-5EX-NHS (2.5 mg, 4.23 µmol). The reaction was allowed to stir at room temperature overnight. The reaction was monitored via HPLC using the method in Table 5.

**TABLE 5**

| Time (min) | Flow Rate (ml/min) | 0.5 M H₃PO₄ (pH 3.0 w/NH₃OH) | 9:1 CH₃CN:H₂0 |
|---|---|---|---|
| 0.0 | 2.0 | 90.0 | 10.0 |
| 2.0 | 2.0 | 90.0 | 10.0 |
| 10.0 | 2.0 | 65.0 | 35.0 |
| 15.0 | 2.0 | 5.0 | 95.0 |
| 28 | 2.0 | 90.0 | 10.0 |

The reaction proceeded very quickly initially forming the desired product after only 10 minutes of contact. CBC-208 has a retention time of 11.47 minutes and the product (CBC-123) has a retention time of 14.24 minutes. With the addition of another equivalent of the fluorescein compound the reaction goes to completion and the crude mixture is 88% pure.

HPLC analysis of the starting material fluorescein-5EX-NHS shows that it is only 75% pure, which explains why an additional equivalent was necessary in order to drive the reaction to completion.

CBC-123 (b-fluorescein cobalamin derivative): This compound is nearly 90% pure as the crude isolate from the synthesis, with the majority of the impurity being unreacted CBC-208. A positive-ion electrospray mass spectrum (ES⁺) was obtained of the crude material showing a M+1 peak (2013) and the corresponding M+Na peak (2035). The yield before purification is 22%.

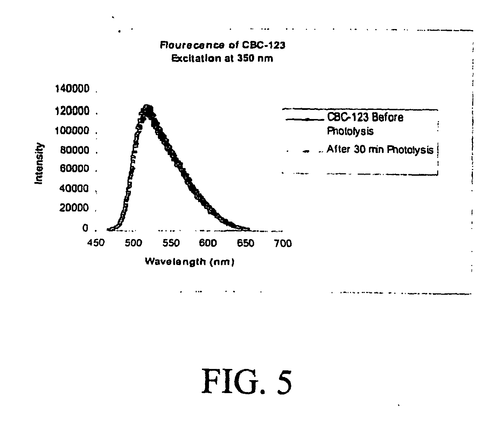

A fluorescence spectrum of this compound was taken of the crude compound before and after photolysis with excitation at 350 nm (see Figure 5). There is no significant change in fluorescence before and after photolysis suggesting that the compound is photostable and is overtly fluorescent and does not exhibit diminished fluorescence from the proximity of cobalamin.

### EXAMPLE 6

### Ex vivo Examination of Breast Tumor Tissue via Microscopy

Samples of malignant and benign tumors, including tumors of the breast, with attached normal margin tissue are excised from patients. These samples are taken with approval of the University of Utah Institutional Review Board (IRB) and the Huntsman Cancer Institute Clinical Cancer Investigation Committee (CCIC). The live tissue samples are incubated with one of the fluorescent cobalamin derivatives prepared above for 4-6 hours. Thin tissue sections of each sample are prepared with a cryomicrotome and the amount of fluorescent marker is quantified in normal and cancerous tissue by epifluorescence microscopy. Corresponding tissue sections are stained with hematoxylin/eosin (H&E) stain for evaluation by an anatomical pathologist. The interface between normal and cancerous cells is examined carefully. Cells from the interior of the tumor are also examined for uptake of fluorescent marker, since cells within hypoxic regions of a tumor often have decreased metabolism.

More specifically, Minimum Essential Medium, alpha modification (α-MEM; 7.5% newborn calf serum, 2.5% fetal bovine serum, 0.2% nystatin, 2.5% penicillin/streptomycin, pH7.2; Sigma) was prepared and aliquoted (10 mL) into sterile 25 mL screw top tissue culture flasks. The media was brought to 37°C, and tissue samples were incubated with fluorescently labeled cobalamins (50 nM; cobalamin-Oregon Green and cobalamin-naphthofluorescein conjugates of Example 1 and cobalamin-fluorescein conjugate of Example 5) and recombinant human TCII (50 pM) in α-MEM for 3 hours. Human breast tissue samples were procured under an IRM-approved protocol. The tissue was removed from the flask, washed with Dulbecco's Phosphate Buffered Saline (DPBS; Sigma), and mounted on a brass plate at -20°C with OCT compound (Shandon) for frozen section slicing. Tissue was sliced (4-6 µm sections) in a CTD Harris cryostat at -20°C. Thin tissue sections were pulled back with a small artist brush and fixed to a microscope slide with 100% ethanol. Slides were stained using a standard hematoxylin staining procedure: 95% ethanol, 20 seconds; water, 5 seconds; hematoxylin (Fisher), 45 seconds; water, 5 seconds; bluing solution (tap water), 10 seconds; 95% ethanol, 10 seconds; 100% ethanol, 10 seconds; xylene, 10 seconds; and xylene, 10 seconds. Slides were evaluated by phase contrast and epifluorescence microscopy at 10x, 60x and 100x magnification.

3-[4,5-Dimethylthiazol-2-yl]-2,5-diphenyltetrazolium thiazolyl bromide (MTT; Sigma) was used to qualitatively determine the metabolic competency of the tissue after 3 hours incubation time with fluorescent cobalamin. A portion of the tissue was removed from the media, washed with DPBS, and immersed in MTT (2 mL; 2.5 mg/mL). This tissue was incubated for 3 hours under a 5% CO₂ atmosphere at 37°C. During this incubation period, viable cells in the tissue sample reduced the MTT dye to purple formazan by succinate dehydrogenase activity (Cells and Celis, 1998). The tissue was washed with DPBS and prepared according to the cryomicrotome procedure outlined above to ensure the metabolic competency of the tissue.

The fluorescent cobalamin bioconjugates accumulated to some extent in both neoplastic and healthy breast tissue, with the neoplastic breast tissue sequestering more fluorescent cobalamin than healthy breast tissue. The amount of fluorescent cobalamin sequestered by healthy breast tissue is larger than expected, but it is believed that it is due to non-specific binding to structures within connective tissue rather than to significant internalization by healthy cells.

### EXAMPLE 7

### Ex vivo Examination of Cancer Cells in Lymph Nodes

Excised lymph nodes with metastatic disease are removed from patients and incubated for 4-8 hours with one of the fluorescent cobalamin derivatives prepared above. Each lymph node is sectioned and examined microscopically for transport of the fluorescent cobalamin into cancer cells. This experiment showed the ability of metastatic cells within lymph nodes to take up sufficient fluorescent cobalamin for imaging and visualization.

### EXAMPLE 8

### Use of Fluorescent Cobalamin to determine whether a patient will respond favorably to chemotherapy with a cobalamin-based therapeutic bioconjugate

A bone marrow aspirate or a peripheral blood sample from a patient with leukemia is incubated with a fluorescent cobalamin conjugate. After 4-8 hours, bone marrow aspirate or peripheral blood sample is washed to remove unincorporated fluorescent label and the cell sample subjected to qualitative or quantitative fluorescence analysis by epifluorescence microscopy or flow cytometry. Cells that have taken up a significant amount of fluorescent cobalamin exhibit a brighter fluorescence. The uptake of a significant amount of fluorescent cobalamin indicates that the type of leukemia the patient has will respond favorably to treatment with a cobalamin-based therapeutic. A bone marrow aspirate or a peripheral blood sample that does not show significant fluorescence after treatment with a fluorescent cobalamin conjugate indicates that the patient will not respond favorably to a cobalamin-based therapeutic conjugate. A similar approach can be applied to solid tumors. In this case, a portion of the excised tumor tissue is incubated with the fluorescent cobalamin conjugate and, after about 4-8 hours, fluorescence in the tumor tissue is quantified. The greater fluorescence exhibited by the tumor tissue, the greater the likelihood that the cancer will respond favorably to treatment with a cobalamin-based chemotherapeutic.

### EXAMPLE 9

### Synthesis of CobalaFluor Y

*General Desalting Procedure.* All cobalamins were desalted with a 10 g C-18 SepPak (Waters, Inc.) by conditioning the cartridge with two column volumes of methanol and three column volumes of deionized water. The cobalamin was applied to the column, washed with three column volumes of deionized water, and eluted with methanol (10 mL). The methanol was removed via rotary evaporation and the product was dried by lyophylization.

*Preparation of cyanocobalamin-b-monocarboxylic acid.* Cyanocobalamin-*b*-monocarboxylic acid was prepared according to a modified published protocol (Anton et al., 1980). In brief, CNCb1 (3.5 g, 2.6 mmol) was dissolved in 350 mL of 1.0 M HCI. The reaction was heated to 37° C for 4 hours and monitored via reverse phase HPLC. The crude material was desalted and could then be purified via semi-prep HPLC. However, since the crude reaction mixture contained over 45% cyanocobalamin (via HPLC) an ion exchange column was used to separate the unreacted cyanocobalamin. Crude material was dissolved in ddH2O and applied to a 2.5 x 30 cm Dowex AG-X1 (acetate form) column. CNCb1 was eluted from the column with deionized water. The three monocarboxylic acids were then eluted with 0.04 M sodium acetate (pH 4) and were further purified via semi-preparative HPLC. The b-monocarboxylic acid was isolated (10% overall yield) in 97 % purity by analytical HPLC; ES⁺ MS: (1:1 H₂O:CH₃CN) M+H =1356.3 (calc. C₆₃H₈₈CoN₁₃O₁₅P = 1356.5), M+Na+ = 1378.4 (calc. C₆₃H₈₈CoN₁₃O₁₅PNa= 1378.5). Both the d- and e-monocarboxylic acids were also isolated in 4% and 7% overall yields respectively.

*Analytical HPLC method for cyanocobalamin-b-monocarboxylic acid:* Analytical chromatography was carried out at a flow rate of 2 mL/min using a Waters DeltaPak C-18 300 x 3.9 mm column. After an initial 2 minute isocratic flow of 90% solution A (0.05 M phosphate buffer, pH 3.0) and 10% solution B (9:1 acetonitrile and water), a 16 minute linear gradient to 83.7% A and 16.3% B eluted the desired b-monocarboxylic derivative with a retention time of 15.7 minutes. The d-monocarboxylic acid had a retention time of 16.9 minutes and the e-monocarboxylic acid had a retention time of 19.5 minutes.

*Semi-preparative HPLC for cyanocobalamin-b-monocarboxylic acid:* Chromatography was carried out at a flow rate of 40 mL/min using a Waters DeltaPak C-18 2.5 x 30 cm semi-preparative column. After a 4.1 minute isocratic flow of 90% solution A (0.05 M phosphate buffer pH 3.0) and 10% solution B (9:1 acetonitrile and water), a 32.9 minute linear gradient to 83.7% A and 16.3% B eluted the cobalamin derivative. The retention times of the three CNCb1-monocarboxylic acids were as follows: the b-monocarboxylic acid eluted at 23.1 minutes, the d-monocarboxylic acid at 26.6 minutes and the e-monocarboxylic acid at 32.1 minutes.

*Synthesis of cyanocobalamin-b-(5-aminopentylamide).* Cyanocobalamin-b-monocarboxylic acid 1 (50 mg, 0.037 mmol) was dissolved in a dry 10 mL round bottom flask with EDCI (71 mg, 0.37 mmol) and NHS (25 mg, 0.22 mmol). The flask was degassed by flushing with nitrogen for 5 minutes. Dimethylsulfoxide (5 mL) was added via syringe and the reaction mixture stirred for 6 hours. This mixture was removed from the round bottom flask using a gas-tight syringe, and 1,5-diaminopentane (43 µL, 0.37 mmol) was placed in the flask. The Cbl mixture was added dropwise to the 1,5-diaminopentane over a period of 5 minutes to minimize formation of 2: 1 adduct. Reverse phase HPLC was used to monitor the reaction. When starting material was consumed, a solution of 1:1 CH₂Cl₂:diethylether (60 mL) precipitated the cobalamins. The resultant solid was filtered on a medium frit filter, washed with diethylether (2x10 mL), and eluted from the filter with methanol. The crude mixture was diluted with an equal volume of water and injected onto a semi-preparative column to purify the cyanocobalamin-b-(5-aminopentylamide) 2. A fraction containing the desired product was desalted as described above and dried by rotary evaporation. Cyanocobalamin-b-(5-aminopentylamide) was obtained: 70% yield; 98% pure by analytical HPLC; ES⁺ MS: (1:1 H₂O:CH₃CN) M+H = 1440.5 (calc. C₆₈H₁₀₀CoN₁₅O₁₄P = 1440.7), M+Na⁺ = 1462.4 (calc. C₆₈H₁₀₀CoN₁₅O₁₄PNa = 1462.6); ε₃₆₂ₘₘ = 19500 M⁻¹cm⁻¹ in H₂O.

*Analytical HPLC method for cyanocobalamin-b-(5-aminopentylamide) 2:* Analytical chromatography was carried out at a flow rate of 2 mL/min on a Waters DeltaPak C-18 300 x 3:9 mm column. After a 2 minute isocratic flow of 95% solution A (0.05 M phosphate buffer, pH 3.0) and 5% solution B (9:1 acetonitrile and water), a 16.4 minute linear gradient to 70% A and 30% B eluted the compound of interest at 11.8 minutes.

*Semi-preparative HPLC for cyanocobalamin-b-(5-aminopentylamide) 2:* Semi-preparative chromatography was carried out at 40 mL/min using a Waters DeltaPak C-18 25 x 30 cm semi-preparative column. After an isocratic flow of 95% solution A (0.05 M phosphate buffer pH 3.0) and 5% solution B (9:1 acetonitrile and water) for 4.1 minutes, an 18 minute linear gradient to 70% A and 30% B eluted the desired product.

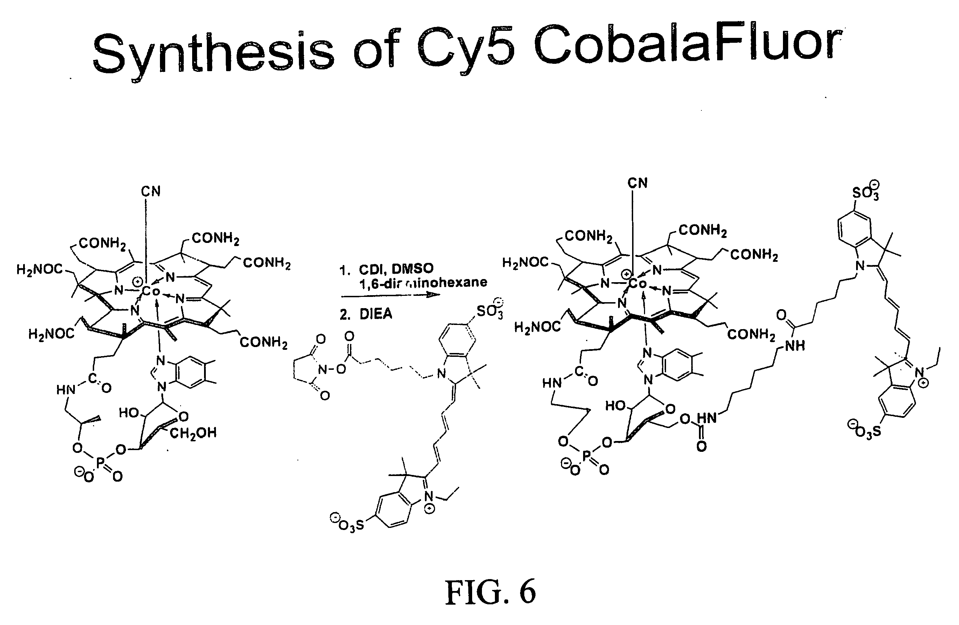

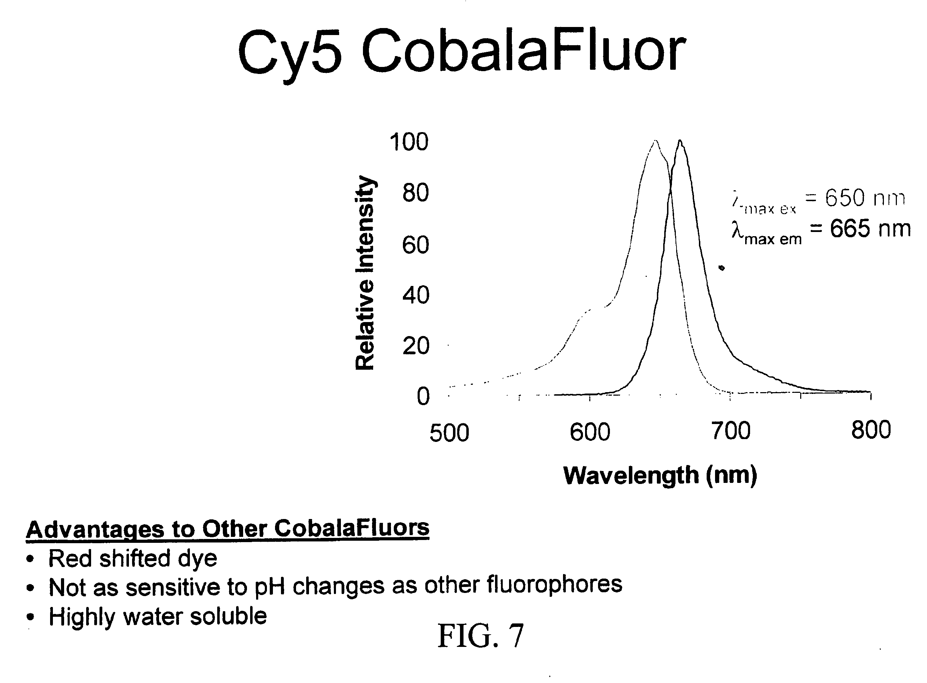

*Synthesis of CobalaFluor Y (Cy5-Cobalamin = Cy5-Cbl = Cy5 CobalaFluor).* This synthesis is shown in Figure 6. Briefly, cyanocobalamin-ribose-5'-0-(6-aminohexylamide) was prepared using cyanocobalamin (Sigma Chemical Co.) according to a published protocol (McEwan et al., 1999). Cobalamins were precipitated using 2:1 diethylether:methylene chloride (50 mL) and also washed with this solvent mixture (2x10 mL). The reaction was monitored and the product purified via reverse phase HPLC. The product was desalted according to standard procedure. Cyanocobalamin-ribose5'-0-(6-aminohexylamide) (20 mg, 0.013 mmol) was placed in a dry 10 mL round bottom flask and degassed by flushing with nitrogen for 5 minutes Dimethylsulfoxide (1 mL) was added via syringe to dissolve the cobalamin. Cy5 succinimidyl ester (10 mg, 0.013 mmol; Amersham Pharmacia) and DIPEA (15 µL, 0.13) were added to the flask and the reaction mixture stirred for 1 hour. Reverse phase HPLC was used to monitor the reaction. When starting material was consumed, a solution of 2:1 diethylether:CH₂Cl₂ (50 mL) precipitated the cobalamins. The resultant solid was filtered on a fine frit filter, washed with the diethylether and CH₂Cl₂ mixture (2x10 mL), and eluted from the filter with methanol. The crude mixture was injected onto a semi-preparative column to purify CobalaFluor Y and desalted according to standard procedure. Figure 7 shows fluorescence emission spectrum of CobalaFluor Y.

*Analytical HPLC method for cyanocobalamin-ribose-5'-0-(6-aminohexylamide):* Analytical chromatography was carried out at a flow rate of 2 mL/min using a Waters DeltaPak C-18 300 x 3.9 mm column. After an initial 2 minute isocratic flow of 95% solution A (0.05 M phosphate buffer, pH 3.0) and 5% solution B (9:1 acetonitrile and water), an 18 minute linear gradient to 70% A and 30% B eluted the desired cyanocobalamin-ribose-5'-0-(6-aminohexylamide) with a retention time of 12.5 minutes.

*Semi-preparative HPLCfor cyanocobalamin-ribose-5'-0-(6-aminohexylamide) :* Chromatography was carried out at a flow rate of 40 mL/min using a Waters DeltaPak C-18 2.5 x 30 cm semi-preparative column. After a 4.1 minute isocratic flow of 95% solution A (0.05 M phosphate buffer pH 3.0) and 5% solution B (9:1 acetonitrile and water), a 27.4 minute linear gradient to 70% A and 30% B elated the cobalamin derivative. The retention time of the desired cyanocobalamin-ribose-5'-0- (6-aminohexylamide) was 15.5 minutes.

*Analytical HPLC method for CobalaFluor Y:* Analytical chromatography was carried out at a flow rate of 2 mL/min on a Waters DeltaPak C-18 300 x 3.9 mm column. After a 2 minute isocratic flow of 95% solution A (0.01 M TEA buffer, pH 7.0) and 5% solution B (9:1 acetonitrile and water), a 16.4 minute linear gradient to 45% A and 55% B eluted CobalaFluor Y at 13.6 minutes.

*Semi-preparative HPLC for CobalaFluor Y:* Semi-preparative chromatography was carried out at 20 mL/min using a Waters DeltaPak C-18 25 x 30 cm semi-preparative column. After an isocratic flow of 95% solution A (0.01 M TEA buffer, pH 7.0) and 5% solution B (9:1 acetonitrile and water) for 2 minutes a 27.4 minute linear gradient to 70% A and 30% D eluted the desired product at 12.2 minutes.

### EXAMPLE 10

### Competition Assay

*Materials.* Cobalamins, porcine non-intrinsic factor (50:1 mixture of HC and IF), and porcine intrinsic factor were purchased from Sigma Chemical Co. HPLC traces were obtained using a Waters Delta 600 system equipped with a Waters 2487 dual wavelength absorbance detector. BIACORE 2000 and 3000 (BIACORE AB) instruments were used for surface plasmon resonance biosensor analysis.

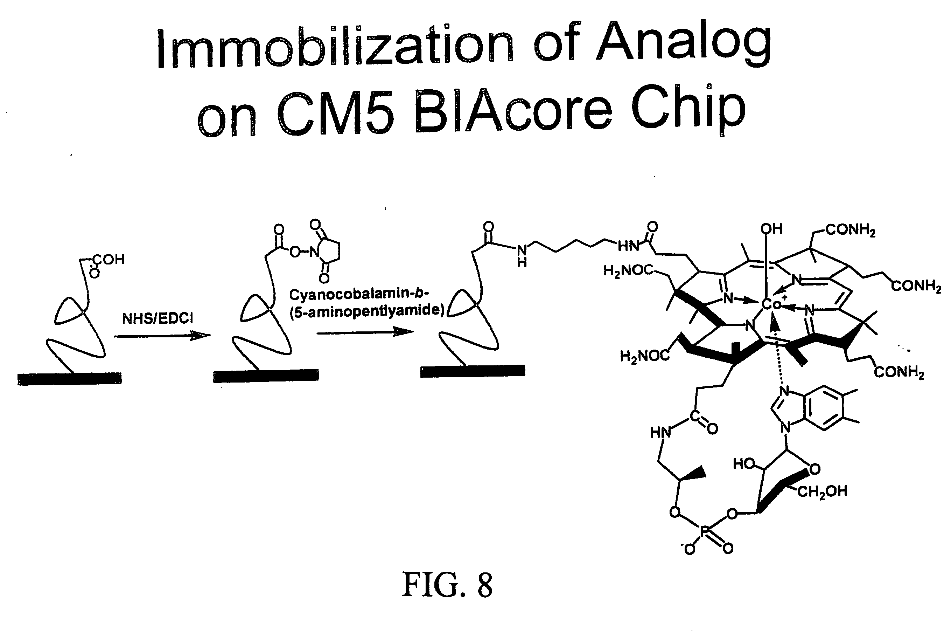

*Immobilization of CNCbl-b-(5-aminopentylamide).* All SPR studies were carried out on a BIACORE 2000 optical biosensor. Carboxymethyl dextran surfaces in the flow cells of a standard CM5 sensor chip (BIACORE AB) were activated by flowing a mixture of 0.1 M EDCI and 0.025 M NHS at 37°C through the chip at 20 µL/min for 15 minutes. CNCbl-b-(5-aminopentylamide) 2, diluted in 10 mM sodium acetate at pH 4.5, was immobilized on three flow cells of the chip as shown in Figure 8. High density sensor surfaces (500-700 RU) were created by pulsing the Cbl analog over the flow cells for 40 minutes at a rate of 2 µL/min. The remaining binding sites on the surface of the chip in all four flow cells were blocked with 1.0 M ethanolamine, pH 8.5, for 16 minutes at 5 µL/min. Flow cell 3 was used as a reference surface to subtract non-specific binding and instrument noise.

*Protein Standard Curve.* All standard curve and competition assays were performed using HBS running buffer (150 mM NaCl, 10 mM HEPES, pH 7.5, 3.4 mM EDTA, 1 mg/mL BSA, and 0.005% P20 surfactant) at 30°C. Calibration curves for rhTCII, NIF, and IF binding CNCb1-b-(5-aminopentylamide) were generated as follows. Stock solutions of each protein (15.6-500 pM) diluted in HBS buffer were injected through the flow cells at 20 µL/min for 10 minutes to analyze binding. The bound protein was removed with 8 M urea, 0.125% SDS, and running buffer. Each protein sample was analyzed in duplicate.

*Determination of the Apparent Solution Equilibrium Dissociation Constants.* The binding of rhTCII, NIF, and IF to various cobalamin analogs were analyzed by a solution competition binding assay (Nieba et al., 1996). Analog concentrations ranging from 0.01-100 nM were incubated in equal volume with 200 pM rhTCII, 200 pM NIF, or 500 pM IF. Binding data were generated by injecting an aliquot of the competing Cbl analog and protein at a rate of 20 µL/min for 10 minutes at 30°C, and the surface was regenerated with pulses of 8 M urea, 0.125% SDS, and buffer. The competition assay for each cobalamin was performed in duplicate.

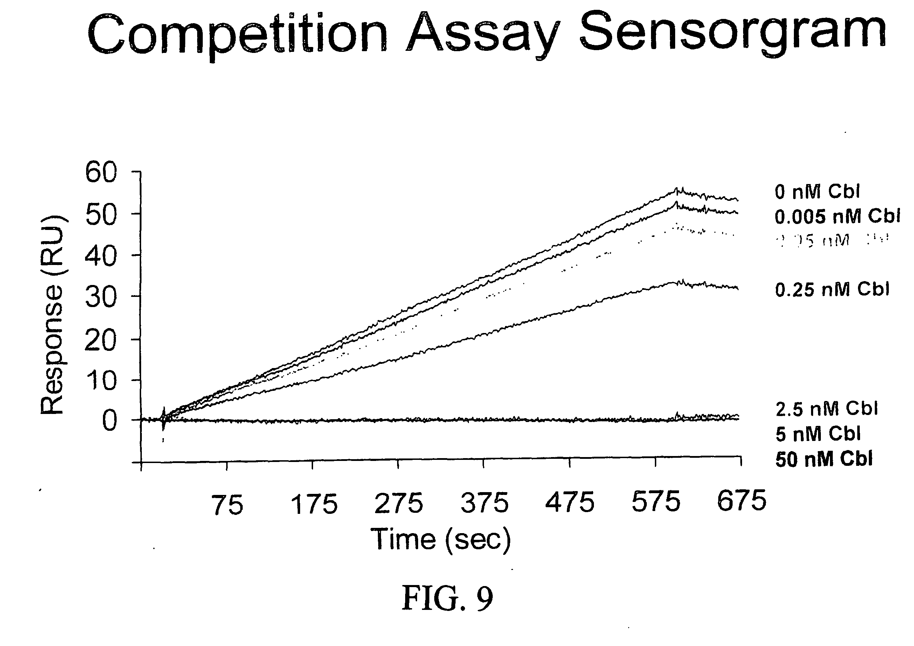

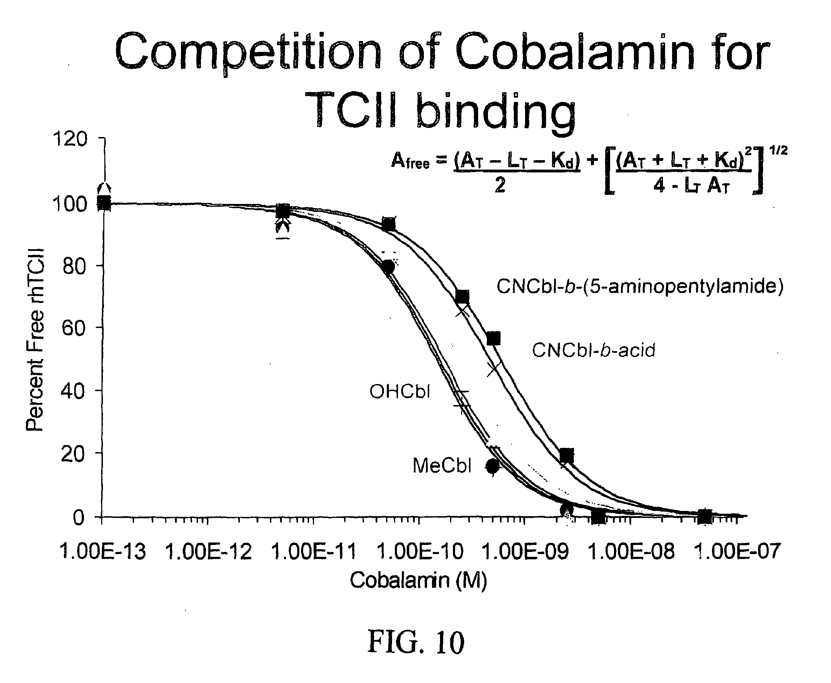

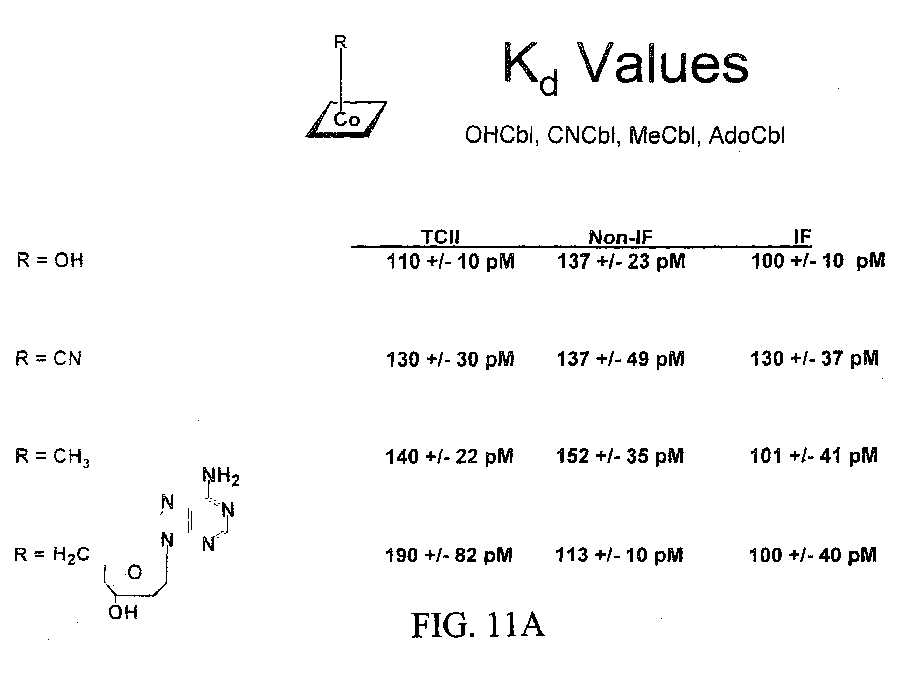

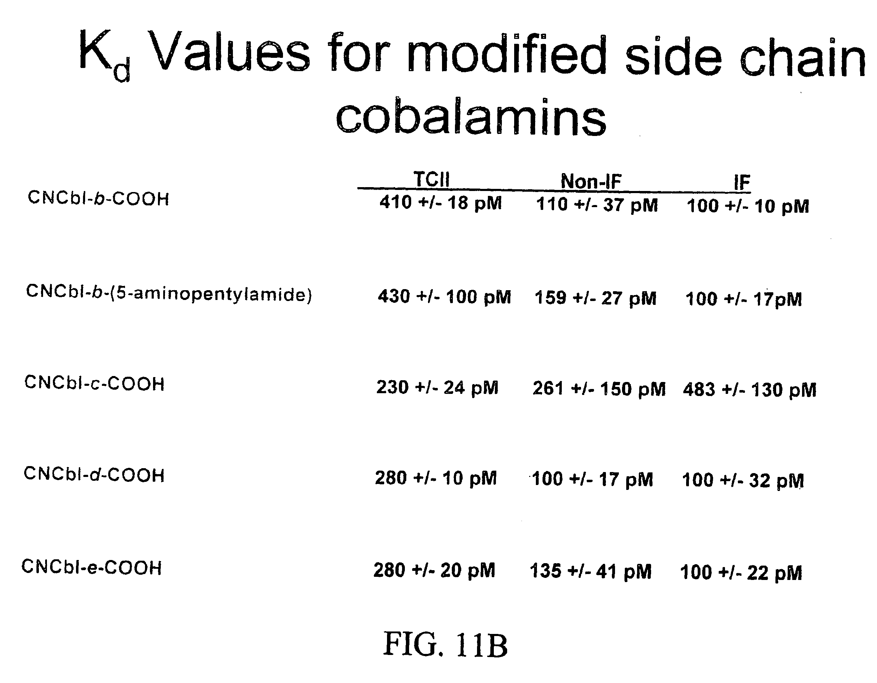

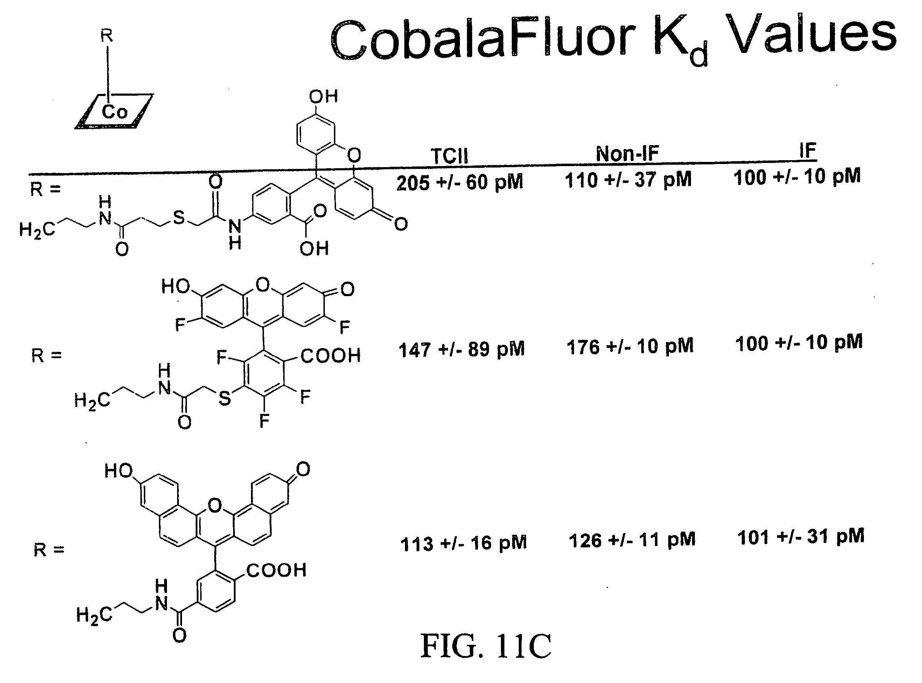

*Data Analysis.* Biosensor data were prepared for analysis by subtracting the binding responses observed from the reference surface and subtracting an average of three blank injections (Myszka, 1999). Data from the competition assays were fitted with non-linear least squares regression analysis supplied with BIAevaluations 3.0 software. Figure 9 shows the competition assay sensogram. Figure 10 shows the competition of cobalamin for TCII binding. The binding data is shown in Figures 11A-11C. These results demonstrate that cobalamin analogs are recognized by cobalamin transport proteins (transcobalamin, haptocorrin and intrinsic factor) with high affinity. This recognition has also been shown by surface plasmon resonance. The attachment of large molecules to cobalamin does not appear to affect protein binding.

### EXAMPLE 11

### Animal Model Study

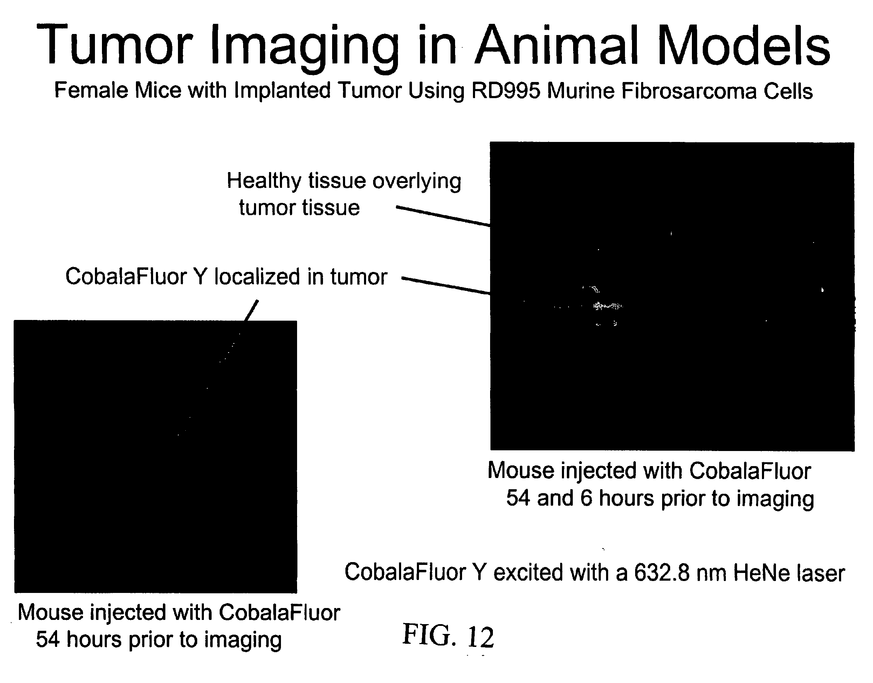

*In Vivo Uptake in Mice with Tumors.* Tumors are implanted in mice by implanting 1x10⁶ RD995 tumor cells subcutaneously on the right hind leg of female mice. The mouse tumor cell line was *propagated in vitro.* Six weeks after implantation of the cells, a 10 mm tumor was visible. At this time, the mice were given a retro-orbital intravenous injection of 2.2 µg of CobalaFluor Y dissolved in sterile saline. At 6 hours post-injection, the mouse was sedated with the inhalation halothane. The tumor was sliced open and irradiated with a 633 nm HeNe laser. A tumor on a mouse was also analyzed at 54 hours post-injection of CobalaFluor Y using the HeNe laser. The mice were dissected so internal organs and healthy tissue could be analyzed. The results are shown in Figure 12, which demonstrates that fluorescently labeled cobalamin localizes in tumor tissue in mice.

### EXAMPLE 12

### Tissue Uptake Study

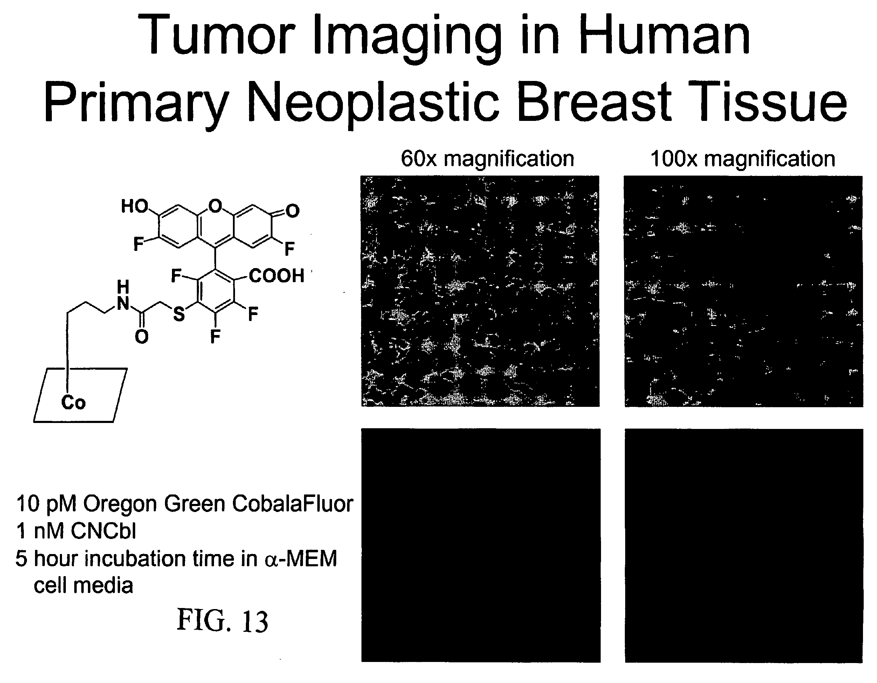

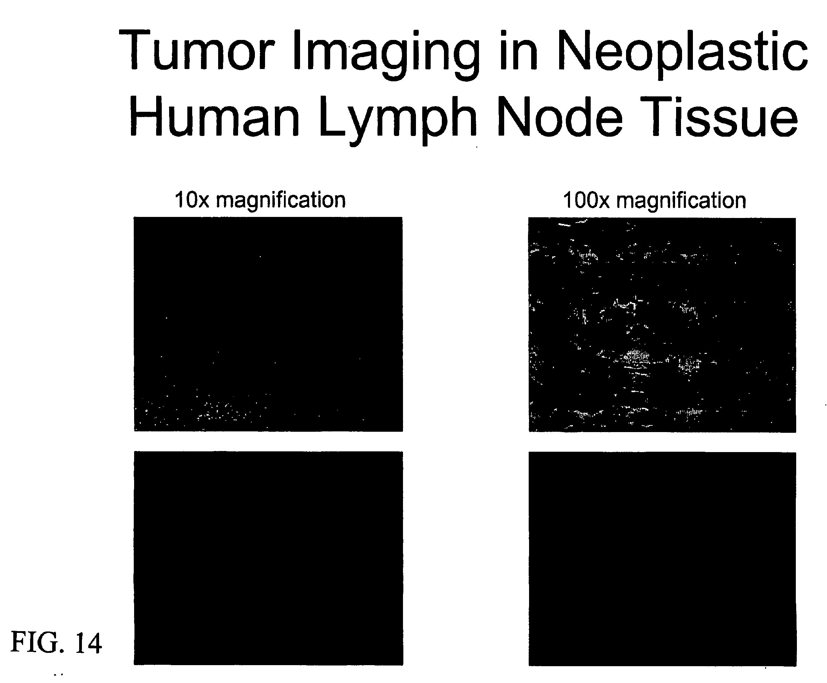

*Fluorescent cobalamin uptake.* Minimum Essential Medium, alpha modification (a-MEM; 7.5 % newborn calf serum, 2.5% fetal bovine serum, 0.2% nystatin, 2.5% penicillin/streptomycin, pH 7.2; Sigma) was prepared and aliquoted (10 mL) into sterile 25 mL as screw top tissue culture flasks. The media was brought to 37°C, and tissue samples (neoplastic breast tissue, healthy breast tissue, neoplastic lymph node tissue and healthy lymph node tissue) were incubated with fluorescently labeled cobalamins (10 pM), cyanocoblamin (1 nM) and in α-MEM for 3 hours. Human tissue samples were procured under an IRB-approved protocol. The tissue was removed from the flask, washed with Dulbecco's Phosphate Buffered Saline (DPBS; Sigma), and mounted on a brass plate at -20°C with OCT compound (Shandon) for frozen section slicing. Tissue was sliced (4-6 11m sections) in a CTD Harris cryostat at -20°C. Thin tissue sections were pulled back with a small artist brush and fixed to a microscope slide with 100% ethanol. Slides were stained using a standard hematoxylin staining procedure: 95% ethanol, 20 seconds; water, 5 seconds; hematoxylin (Fisher), 45 seconds; water, 5 seconds; bluing solution (tap water), 10 seconds; 95% ethanol, 10 seconds; 100% ethanol, 10 seconds; xylene, 10 seconds; and xylene, 10 seconds. Slides were evaluated by phase contrast and epifluorescence microscopy at 10x, 60x, and 100x magnification. Tumor imaging in (a) neoplastic breast tissue is shown in Figure 13 and (b) neoplastic lymph node tissue is shown in Figure 14.

### Cell viability and tissue metabolic activity assay.

3-[4,5-Dimethylthiazol-2-yl]-2,5-diphenyltetrazolium thiazolyl bromide (MTT; Sigma) was used to qualitatively determine the metabolic competency of the tissue after 3 hours incubation time with fluorescent cobalamin. A portion of the tissue was removed from the media, washed with DPBS, and immersed in MTT (2 mL; 2.5 mg/mL). This tissue was incubated for 3 hours under a 5% CO2 atmosphere at 37°C. During this incubation period, viable cells in the tissue sample reduced the MTT dye to purple formazan by succinate dehydrogenase activity. The tissue was washed with DPBS and prepared according to the cryomicrotome procedure outlined above to ensure the metabolic competency of the tissue. It was found that in vitro both healthy and neoplastic tissue take up fluorescent cobalamins.

### EXAMPLE 13

### Use of surgical telescopic device in pig surgery

A surgical use of the device for identifying lymph tissue that drains an anatomical region and/or identifying malignant cells was illustrated in a pig surgery. The inguinal node area was used due to familiarity with the anatomy from a previous open dissection. A 10 mm trocar was inserted into the subcutaneous layer of the groin and the region was insufflated with carbon dioxide at a pressure of 15 mm Hg. Two 5 mm trocars were inserted for dissection instruments. The lymph node region and the route of the principal lymphatic drainage from the lower extremity were exposed using sharp and blunt dissection with the laparoscope shown in FIGS. 15-18. CobalaFluor Y (1.5 mg/mL in a one mL volume) was injected into the subcutaneous layer near the principal lymphatic drainage. CobalaFluor Y was identified within 2 to 5 minutes with the laparoscope shown in FIGS. 15-18 in the lymphatic trunks and the sentinel node visually by white light and by fluorescence stimulated by red laser light having a wavelength of 633 nm.

### EXAMPLE 14

### Use of device in other surgeries

The disclosed method and/or apparatus may be used similarly to that described in Example 13 in a variety of surgeries to identify lymph nodes and vessels that drain a particular anatomical region and/or neoplastic tissue that has not been surgically removed. In particular, the field of interest may be an excised area, such as tissue from which a neoplasm is to be or has been removed. For example, the anatomical region of interest may be injected or infused with the fluorescent cobalamin to identify the tumor and/or lymph nodes draining the *situs* of the tumor. The amount of injected fluorescent cobalamin can vary widely, but one particular range is about 10 mL/min to about 100 mL/min. The tumor and/or lymph nodes are then excised following known surgical procedures, and a surgical telescope (such as one of the devices disclosed in this specification) is positioned for magnified inspection of the tumor micromargins. Since the fluorescent cobalamin has a selective affinity for neoplastic cells, the presence of the fluorescent cobalamin in the surgical margins of the excised tissue indicates that additional marginal tissue should be removed, until the presence of fluorescing tissue or cells is no longer detected.

It will be appreciated that the methods and compositions of the instant disclosure can be incorporated in the form of a variety of embodiments, only a few of which are disclosed herein. It will be apparent to the artisan that other embodiments exist. Thus, the described embodiments are illustrative and should not be construed as restrictive.

### LIST OF REFERENCES

Cannel, R. (1975). "Extreme Elevation of Serum Transcobalamin I in Patients with Metastatic Cancer." New Engl. J. Med. 292:282-284.
Celis, A. and Celis, J.E. (1998). Cell Biology, pp. 9-11*.*
Collins, D. A. and Hogenkamp, H. P. C. (1997). "Transcobalamin II Receptor Imaging via Radiolabeled Diethylene-Triaminepentaacetate Cobalamin Analogs." J. Nucl. Med. 38:717-723.
Collins, D.A. et al. (1999). "Tumor Imaging via Indium-111-Labeled DTPA-Adenosylcobalamin." Mayo Clinic Proceedings 74: 687-691.
Collins, D.A. et al. (2000). "Biodistribution of Radiolabeled Adenosylcobalamin in Patients Diagnosed with Various Malignancies." Mayo ClinicProceedings 75:568-580.
Flodh, H. (1968). "Accumulation of labelled Vitamin B-12 in Some Transplanted Tumors." Acta Ratio/. Supp/. 284:55-60.
Hogenkamp, H. P.C., et al. (1999). "The Pharmacological Uses of Cobalamin Bioconjugates." In The Chemistry and Biochemistry of B-12, Banerjee, R., Ed., John Wiley & Sons, New York, pp. 385-410.
Howard, W.A. et al. (1997). "Sonolysis Promotes Indirect C-Co Bond Cleavage of Alkylcob(III)alamins." Bioconj. Chem. 8:498-502.
McGreevy, J. M. (1998). "Sentinel Lymph Node Biopsy in Breast Cancer." Curr. Surg 55:301-4.
Mitchell, A. M. et al. (1999). "Targeting Leukemia Cells with Cobalamin Bioconjugates" In Enzymatic Mechanisms, Frey, P. A.; Northrop, D. B., Eds., pp 150-154.
McMasters, K. M. et al. (1999). "Sentinel Lymph Node Biopsy for Breast CancerNot yet the Standard of Care." New England J. Med. 339: 990*.*
Morton, D. L. et al. (1992). "Technical Details of Intraoperative Lymphatic Mapping for Early Stage Melanoma." Arch. Surg 127:392-9.
Rachmilewitz, B. et al. (1971)., "Serum Transcobalamin in Myeloid Leukemia." J. Lab. Clin. Med. 78:275.
Schneider, Z. and Stroinski, A. (1987). Comprehensive B12, de Grnyter, Berlin, pp. 358.

## Claims

1. An apparatus for detecting and imaging fluorescent, phosphorescent or luminescent material in a host, comprising:
(a) a light-generating source (12) that illuminates the fluorescent, phosphorescent or luminescent material, wherein the light-generating source includes a red-light-generating laser or a laser diode, and further includes a white light source;
(b) a surgical telescopic device (10) having a distal end (14) and a proximal end (15);
(c) a focusing lens (22) having a first end positioned adjacent to the proximate end of the surgical telescopic device;
(d) a camera (11) coupled to a second end of the focusing lens;
(e) at least one member (18) interposed between the proximal end of the surgical telescopic device and the camera, wherein the member removes substantially only the wavelength transmitted by the red-light-generating laser or laser diode; **characterized in that** the apparatus further comprises
(f) a mechanism for switching between the red-light-generating laser or laser diode for fluorescence viewing and the white light source for conventional viewing.

2. The apparatus of claim 1, wherein the member is a holographic notch filter.

3. The apparatus of claim 2, further comprising at least one second filter disposed between the surgical telescopic device and the camera.

4. The apparatus of any of claims 1, 2 or 3, wherein the camera comprises a charge-coupled device camera.

5. The apparatus of claim 2, wherein the red-light-generating laser or laser diode are optically coupled to the surgical telescopic device and wherein the holographic notch filter deflects substantially only the wavelength transmitted by the red-light-generating laser or laser diode.

6. The apparatus of claim 1, wherein the red-light-generating laser or laser diode generates light of a wavelength of about 630 nm to about 635 nm.

7. The apparatus of any of claims 1 to 6, wherein the apparatus comprises a surgical telescopic device having a distal end configured to insert into an incision or opening in the host.

8. The apparatus of claim 1, wherein the light-generating source comprises a dual-lamp xenon source.

9. A kit comprising the apparatus of claim 1, and a fluorescent, phosphorescent or luminescent material comprising a cobalamin, wherein the cobalamin has a structure represented by the general formula 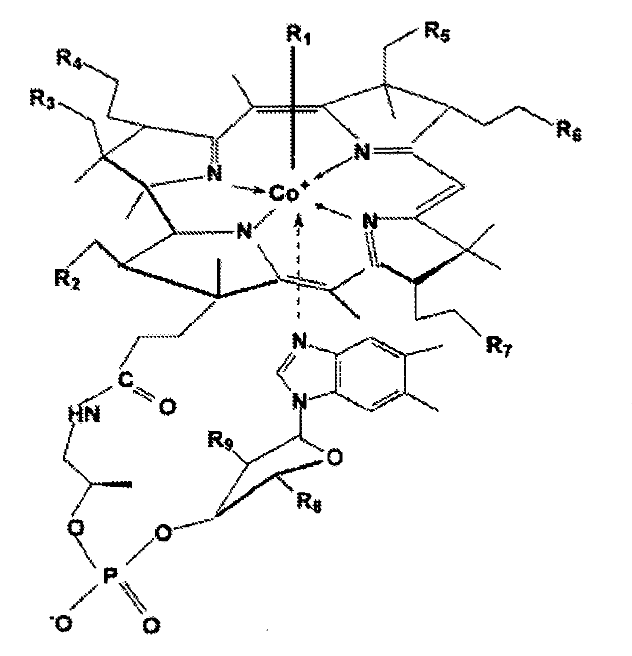 where R₁, is CN, OH, OH₂, CH₃, 5'-deoxyadenosine or (CH₂)ₚNHC(=S)Y; R₂, R₃, R₄, R₅, R₆, and R₇ are independently CONH₂ or CO-XₘY; R₈ is CH₂OH, O(C=O)XₘY or CH₂O(C=O)XₘY; R₉ is OH or O(C=O)XₘY; X is a linker having the formula N(CH₂)ₙNHO(C=O) or NH-(CH₂)ₙ-NH; Y is a fluorophore, a phosphorophore, or a chemiluminescent chromophore; m is 0 or 1, n is 0-50 and p is 2-10, with the proviso that at least one of R₁ - R₉ groups contains Y.

## Patentansprüche

1. Vorrichtung zum Erfassen und Abbilden von fluoreszierendem, phosphoreszierendem oder lumineszierendem Material in einem Wirt, umfassend:
(a) eine Lichterzeugungsquelle (12), die das fluoreszierende, phosphoreszierende oder lumineszierende Material beleuchtet, wobei die Lichterzeugungsquelle einen rotes Licht erzeugenden Laser oder eine rotes Licht erzeugende Laserdiode einschließt und weiterhin eine Weißlichtquelle beinhaltet;
(b) eine chirurgische Teleskopvorrichtung (10) mit einem distalen Ende (14) und einem proximalen Ende (15);
(c) eine Fokussierungslinse (22) mit einem ersten Ende, das neben dem benachbarten Ende der chirurgischen Teleskopvorrichtung positioniert ist;
(d) eine Kamera (11), die mit einem zweiten Ende der Fokussierungslinse gekoppelt ist;
(e) zumindest ein Element (18), das zwischen dem proximalen Ende der chirurgischen Teleskopvorrichtung und der Kamera eingefügt ist, wobei das Element im Wesentlichen nur die Wellenlänge entfernt, die von dem rotes Licht erzeugenden Laser oder der rotes Licht erzeugenden Laserdiode übertragen wird; **dadurch gekennzeichnet, dass** die Vorrichtung weiterhin Folgendes umfasst:
(f) einen Mechanismus zum Umschalten zwischen dem rotes Licht erzeugenden Laser oder der rotes Licht erzeugenden Laserdiode für eine Fluoreszenzbetrachtung und der Weißlichtquelle für eine konventionelle Betrachtung.

2. Vorrichtung nach Anspruch 1, wobei das Element ein holografisches Kerbfilter ist.

3. Vorrichtung nach Anspruch 2, weiterhin mit zumindest einem zweiten Filter, das zwischen der chirurgischen Teleskopvorrichtung und der Kamera angeordnet ist.

4. Vorrichtung nach irgendeinem der Ansprüche 1, 2 oder 3, wobei die Kamera eine Ladungskoppelelement-Kamera umfasst.

5. Vorrichtung nach Anspruch 2, wobei der rotes Licht erzeugende Laser oder die rotes Licht erzeugende Laserdiode mit der chirurgischen Teleskopvorrichtung optisch gekoppelt ist und wobei das holografische Kerbfilter im Wesentlichen nur die Wellenlänge ablenkt, die von dem rotes Licht erzeugenden Laser oder der rotes Licht erzeugenden Laserdiode übertragen wird.

6. Vorrichtung nach Anspruch 1, wobei der rotes Licht erzeugende Laser oder die rotes Licht erzeugende Laserdiode Licht mit einer Wellenlänge von ungefähr 630 nm bis ungefähr 635 nm erzeugt.

7. Vorrichtung nach irgendeinem der Ansprüche 1 bis 6, wobei die Vorrichtung eine chirurgische teleskopische Vorrichtung mit einem distalen Ende umfasst, das dazu konfiguriert ist, in einen Einschnitt oder eine Öffnung in dem Wirt eingeführt zu werden.

8. Vorrichtung nach Anspruch 1, wobei die Lichterzeugungsquelle eine Duallampen-Xenonquelle umfasst.

9. Kit mit der Vorrichtung nach Anspruch 1 und einem fluoreszierenden, phosphoreszierenden oder lumineszierenden Material, das ein Cobalamin umfasst, wobei das Cobalamin eine Struktur aufweist, die von der allgemeinen Formel 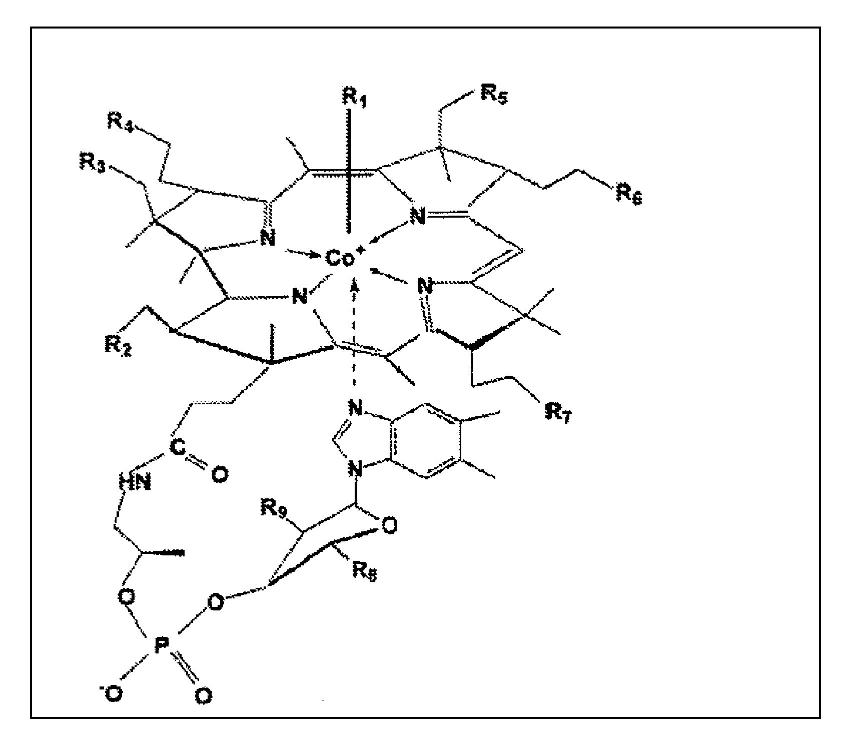 dargestellt wird, wobei R₁ CN, OH, OH₂, CH₃, 5'-Desoxyadenosin oder (CH₂)_{P}NHC(=S)Y ist; R₂, R₃, R₄, R₅, R₆ und R₇ unabhängig CONH₂ oder CO-XₘY sind; R₈ CH₂OH, O(C=O)XₘY oder CH₂O(C=O) XₘY ist; R₉ OH oder O(C=O)XₘY ist; X ein Linker mit der Formel N(CH₂)ₙNHO(C=O) oder NH-(CH₂)ₙ-NH ist; Y ein Fluorophor, ein Phosphorophor oder ein chemilumineszierender Chromophor ist; m 0 oder 1, n 0-50 und p 2-10 ist, mit der Maßgabe, dass von den Gruppen R₁ bis R₉ mindestens eine Y enthält.

## Revendications

1. Appareil pour détecter et imager une matière fluorescente, phosphorescente ou luminescente dans un hôte, comprenant :
a. une source génératrice de lumière (12) qui éclaire la matière fluorescente, phosphorescente ou luminescente, où la source génératrice de lumière comprend une diode laser ou un laser générateur de lumière rouge, et qui comprend en outre une source de lumière blanche :
b. un dispositif chirurgical télescopique (10) doté d'une extrémité distale (14) et d'une extrémité proximale (15) ;
c. une lentille de focalisation (22) munie d'une première extrémité en position adjacente à l'extrémité proximale du dispositif chirurgical télescopique ;
d. une caméra (11) couplée à une deuxième extrémité de la lentille de focalisation ;
e. au moins un élément (18) interposé entre l'extrémité proximale du dispositif chirurgical télescopique et la caméra, où l'élément ne retire sensiblement que la longueur d'onde transmise par la diode laser ou le laser générateur de lumière rouge ; **caractérisé en ce que** l'appareil comprend en outre
f. un mécanisme destiné à commuter entre la diode laser ou le laser générateur de lumière rouge pour visualiser la fluorescence, et la source de lumière blanche pour la visualisation normale.

2. Appareil conforme à la revendication 1, où l'élément est un filtre coupe-bande holographique.

3. Appareil conforme à la revendication 2, comprenant en outre au moins un deuxième filtre placé entre le dispositif chirurgical télescopique et la caméra.

4. Appareil conforme à une quelconque des revendications 1, 2 ou 3, où la caméra comprend une caméra à dispositif de couplage de charge.

5. Appareil conforme à la revendication 2, où la diode laser ou le laser générateur de lumière rouge sont dotés d'un couplage optique avec le dispositif chirurgical télescopique et où le filtre coupe-bande holographique ne défléchit sensiblement que la longueur d'onde émise par la diode laser ou le laser générateur de lumière rouge.

6. Appareil conforme à la revendication 1, où la diode laser ou le laser générateur de lumière rouge génère une lumière dont la longueur d'onde est d'environ 630 nm à environ 635 nm.

7. Appareil conforme à une quelconque des revendications 1 à 6, où l'appareil comprend un dispositif chirurgical télescopique dont l'extrémité distale est configurée pour s'insérer dans une incision ou une ouverture dans l'hôte.

8. Appareil conforme à la revendication 1, où la source lumineuse comprend une source xénon à deux lampes.

9. Ensemble comprenant l'appareil de la revendication 1 et une matière fluorescente, phosphorescente ou luminescente comportant une cobalamine, où la cobalamine est dotée d'une structure représentée par la formule générale 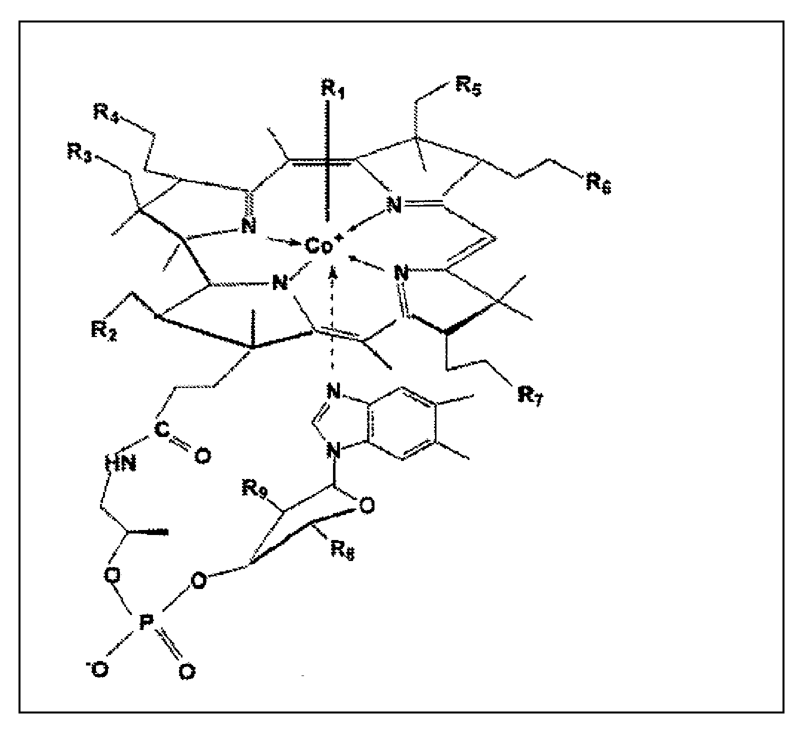 où R₁ est CN, OH, OH₂, CH₃, 5'-désoxyadénosine ou (CH₂)_{P}NHC(=S)Y ; R₂, R₃, R₄, R5, R₆ et R₇ sont indépendamment CONH₂ ou CO-XₘY ; R₈ est CH₂OH, 0(C=0)XₘY ou CH₂0(C=0)XₘY ; R₉ est OH ou 0(C=0)XₘY ; X est un lieur de formule N(CH₂)ₙNH0(C=0) ou NH-(CH₂)ₙ-NH ; Y est un fluorophore, un phosphorophore ou un chromophore chimiluminescent ; m est 0 ou 1, n est 0-50 et p est 2-10, avec la condition qu'au moins un des groupes R₁ - R₉ doit contenir Y.
